# EUROPEAN PATENT APPLICATION

(11) **EP 2 078 455 A1**
(43) Date of publication of application: **15.07.2009**
(21) Application number: 08020953.9
(22) Date of filing: 06.03.2002
(51) Int. Cl.: A01H 5/00, C12N 15/82, C12N 5/10, C12N 9/10, C12P 21/00

(54) **Plant cell having animal-type sugar chain adding function**

(30) Priority: 06.03.2001 JP 2001062704
(62) Divisional of application: 02702772.1
(71) Applicant: The Dow Chemical Company, Midland, MI 48674 (US)
(72) Inventor: Fujiyama, Kazuhito, Suita-shi, Osaka 565-0824 (JP); Seki, Tatsuji, Toyonaka-shi, Osaka 565-0083 (JP)
(74) Representative: Thurgood, Alexander John

(57) **Abstract**

A plant cell having an animal-type sugar chain adding function is provided. The plant cell has an introduced gene encoding an enzyme derived from an animal, and the enzyme can transfer a fucose residue to a reducing terminal acetylglucosamine residue of a sugar chain of a glycoprotein.

SEQUENCE LISTING
<110> Kazuhito FUJIYAMA
<120> Plant cell having animal-type sugar chain adding function
<130> J1-00356138
<140>
<141>
<160> 5
<170> PatentIn Ver. 2.0
<210> 1
<211> 1759
<212> DNA
<213> human
<220>
<221> CDS
<222> (17)..(1744)

<400>
   aaaatctctc tagaaa atg cgg cca tgg act ggt tcc tgg cgt tgg att atg 52
   Met Arg Pro Trp Thr Gly Ser Trp Arg Trp Ile Met
   1 5 10
   ctc att ctt ttt gcc tgg ggg acc ttg ctg ttt tat ata ggt ggt cac 100
   Leu Ile Leu Phe Ala Trp Gly Thr Leu Leu Phe Tyr Ile Gly Gly His
   15 20 25
   ttg gta cga gat aat gac cat cct gat cac tct agc cga gaa ctg tcc 148
   Leu Val Arg Asp Asn Asp His Pro Asp His Ser Ser Arg Glu Leu Ser
   30 35 40
   aag att ctg gca aag ctt gaa cgc tta aaa cag cag aat gaa gac ttg 196
   Lys Ile Leu Ala Lys Leu Glu Arg Leu Lys Gln Gln Asn Glu Asp Leu
   45 50 55 60
   agg cga atg gcc gaa tct ctc cgg ata cca gaa ggc cct att gat cag 244
   Arg Arg Met Ala Glu Ser Leu Arg Ile Pro Glu Gly Pro Ile Asp Gln
   65 70 75
   ggg cca gct ata gga aga gta cgc gtt tta gaa gag cag ctt gtt aag 292
   Gly Pro Ala Ile Gly Arg Val Arg Val Leu Glu Glu Gln Leu Val Lys
   80 85 90
   gcc aaa gaa cag att gaa aat tac aag aaa cag acc aga aat ggt ctg 340
   Ala Lys Glu Gln Ile Glu Asn Tyr Lys Lys Gln Thr Arg Asn Gly Leu
   95 100 105
   ggg aag gat cat gaa atc ctg agg agg agg att gaa aat gga gct aaa    388
   Gly Lys Asp His Glu Ile Leu Arg Arg Arg Ile Glu Asn Gly Ala Lys
       110                 115                 120
   gag ctc tgg ttt ttc cta cag agt gaa ttg aag aaa tta aag aac tta 436
   Glu Leu Trp Phe Phe Leu Gln Ser Glu Leu Lys Lys Leu Lys Asn Leu
   125 130 135 140
   gaa gga aat gaa ctc caa aga cat gca gat gaa ttt ctt ttg gat tta 484
   Glu Gly Asn Glu Leu Gln Arg His Ala Asp Glu Phe Leu Leu Asp Leu
   145 150 155
   gga cat cat gaa agg tct ata atg acg gat cta tac tac ctc agt cag 532
   Gly His His Glu Arg Ser Ile Met Thr Asp Leu Tyr Tyr Leu Ser Gln
   160 165 170
   aca gat gga gca ggt gat tgg cgg gaa aaa gag gcc aaa gat ctg aca 580
   Thr Asp Gly Ala Gly Asp Trp Arg Glu Lys Glu Ala Lys Asp Leu Thr
   175 180 185
   gaa ctg gtt cag cgg aga ata aca tat ctt cag aat ccc aag gac tgc 628
   Glu Leu Val Gln Arg Arg Ile Thr Tyr Leu Gln Asn Pro Lys Asp Cys
   190 195 200
   agc aaa gcc aaa aag ctg gtg tgt aat atc aac aaa ggc tgt ggc tat 676
   Ser Lys Ala Lys Lys Leu Val Cys Asn Ile Asn Lys Gly Cys Gly Tyr
   205 210 215 220
   ggc tgt cag ctc cat cat gtg gtc tac tgc ttc atg att gca tat ggc 724
   Gly Cys Gln Leu His His Val Val Tyr Cys Phe Met Ile Ala Tyr Gly
   225 230 235
   acc cag cga aca ctc atc ttg gaa tct cag aat tgg cgc tat gct act 772
   Thr Gln Arg Thr Leu Ile Leu Glu Ser Gln Asn Trp Arg Tyr Ala Thr
   240 245 250
   ggt gga tgg gag act gta ttt agg cct gta agt gag aca tgc aca gac 820
   Gly Gly Trp Glu Thr Val Phe Arg Pro Val Ser Glu Thr Cys Thr Asp
   255 260 265
   aga tct ggc atc tcc act gga cac tgg tca ggt gaa gtg aag gac aaa 868
   Arg Ser Gly Ile Ser Thr Gly His Trp Ser Gly Glu Val Lys Asp Lys
   270 275 280
   aat gtt caa gtg gtc gag ctt ccc att gta gac agt ctt cat ccc cgt 916
   Asn Val Gln Val Val Glu Leu Pro Ile Val Asp Ser Leu His Pro Arg
   285 290 295 300
   cct cca tat tta ccc ttg gct gta cca gaa gac ctc gca gat cga ctt 964
   Pro Pro Tyr Leu Pro Leu Ala Val Pro Glu Asp Leu Ala Asp Arg Leu
   305 310 315
   gta cga gtg cat ggt gac cct gca gtg tgg tgg gtg tct cag ttt gtc 1012
   Val Arg Val His Gly Asp Pro Ala Val Trp Trp Val Ser Gln Phe Val
   320 325 330
   aaa tac ttg atc cgc cca cag cct tgg cta gaa aaa gaa ata gaa gaa 1060
   Lys Tyr Leu Ile Arg Pro Gln Pro Trp Leu Glu Lys Glu Ile Glu Glu
   335 340 345
   gcc acc aag aag ctt ggc ttc aaa cat cca gtt att gga gtc cat gtc 1108
   Ala Thr Lys Lys Leu Gly Phe Lys His Pro Val Ile Gly Val His Val
   350 355 360
   aga cgc aca gac aaa gtg gga aca gaa gct gcc ttc cat ccc att gaa 1156
   Arg Arg Thr Asp Lys Val Gly Thr Glu Ala Ala Phe His Pro Ile Glu
   365                 370                 375                 380
   gag tac atg gtg cat gtt gaa gaa cat ttt cag ctt ctt gca cgc aga    1204
   Glu Tyr Met Val His Val Glu Glu His Phe Gln Leu Leu Ala Arg Arg
                   385                 390                 395
   atg caa gtg gac aaa aaa aga gtg tat ttg gcc aca gat gac cct tct    1252
   Met Gln Val Asp Lys Lys Arg Val Tyr Leu Ala Thr Asp Asp Pro Ser
               400                 405                 410
   tta tta aag gag gca aaa aca aag tac ccc aat tat gaa ttt att agt    1300
   Leu Leu Lys Glu Ala Lys Thr Lys Tyr Pro Asn Tyr Glu Phe Ile Ser
           415                 420                 425
   gat aac tct att tcc tgg tca gct gga ctg cac aat cga tac aca gaa    1348
   Asp Asn Ser Ile Ser Trp Ser Ala Gly Leu His Asn Arg Tyr Thr Glu
       430                 435                 440
   aat tca ctt cgt gga gtg atc ctg gat ata cat ttt ctc tct cag gca    1396
   Asn Ser Leu Arg Gly Val Ile Leu Asp Ile His Phe Leu Ser Gln Ala
   445                 450                 455                 460
   gac ttc cta gtg tgt act ttt tca tcc cag gtc tgt cga gtt gct tat    1444
   Asp Phe Leu Val Cys Thr Phe Ser Ser Gln Val Cys Arg Val Ala Tyr
                   465                 470                 475
   gaa att atg caa aca cta cat cct gat gcc tct gca aac ttc cat tct    1492
   Glu Ile Met Gln Thr Leu His Pro Asp Ala Ser Ala Asn Phe His Ser
               480                 485                 490
   tta gat gac atc tac tat ttt ggg ggc cag aat gcc cac aat caa att    1540
   Leu Asp Asp Ile Tyr Tyr Phe Gly Gly Gln Asn Ala His Asn Gln Ile
           495                 500                 505
   gcc att tat gct cac caa ccc cga act gca gat gaa att ccc atg gaa    1588
   Ala Ile Tyr Ala His Gln Pro Arg Thr Ala Asp Glu Ile Pro Met Glu
       510                 515                 520
   cct gga gat atc att ggt gtg gct gga aat cat tgg gat ggc tat tct 1636
   Pro Gly Asp Ile Ile Gly Val Ala Gly Asn His Trp Asp Gly Tyr Ser
   525 530 535 540
   aaa ggt gtc aac agg aaa ttg gga agg acg ggc cta tat ccc tcc tac 1684
   Lys Gly Val Asn Arg Lys Leu Gly Arg Thr Gly Leu Tyr Pro Ser Tyr
   545 550 555
   aaa gtt cga gag aag ata gaa acg gtc aag tac ccc aca tat cct gag 1732
   Lys Val Arg Glu Lys Ile Glu Thr Val Lys Tyr Pro Thr Tyr Pro Glu
   560 565 570
   gct gag aaa taa agtcgactca gatgg
   1759
   Ala Glu Lys
   575

<210> 2
<211> 575
<212> PRT
<213> human

<400> 2
Met Arg Pro Trp Thr Gly Ser Trp Arg Trp Ile Met Leu Ile Leu Phe
1               5                   10                  15

Ala Trp Gly Thr Leu Leu Phe Tyr Ile Gly Gly His Leu Val Arg Asp
            20                  25                  30

Asn Asp His Pro Asp His Ser Ser Arg Glu Leu Ser Lys Ile Leu Ala
        35                  40                  45

Lys Leu Glu Arg Leu Lys Gln Gln Asn Glu Asp Leu Arg Arg Met Ala
    50                  55                  60

Glu Ser Leu Arg Ile Pro Glu Gly Pro Ile Asp Gln Gly Pro Ala Ile

65                  70                  75                  80

Gly Arg Val Arg Val Leu Glu Glu Gln Leu Val Lys Ala Lys Glu Gln
                85                  90                  95

Ile Glu Asn Tyr Lys Lys Gln Thr Arg Asn Gly Leu Gly Lys Asp His
            100                 105                 110

Glu Ile Leu Arg Arg Arg Ile Glu Asn Gly Ala Lys Glu Leu Trp Phe
        115                 120                 125

Phe Leu Gln Ser Glu Leu Lys Lys Leu Lys Asn Leu Glu Gly Asn Glu
    130                 135                 140

Leu Gln Arg His Ala Asp Glu Phe Leu Leu Asp Leu Gly His His Glu
145                 150                 155                 160

Arg Ser Ile Met Thr Asp Leu Tyr Tyr Leu Ser Gln Thr Asp Gly Ala
                165                 170                 175

Gly Asp Trp Arg Glu Lys Glu Ala Lys Asp Leu Thr Glu Leu Val Gln
            180                 185                 190

Arg Arg Ile Thr Tyr Leu Gln Asn Pro Lys Asp Cys Ser Lys Ala Lys
        195                 200                 205

Lys Leu Val Cys Asn Ile Asn Lys Gly Cys Gly Tyr Gly Cys Gln Leu
    210                 215                 220

His His Val Val Tyr Cys Phe Met Ile Ala Tyr Gly Thr Gln Arg Thr
225                 230                 235                 240

Leu Ile Leu Glu Ser Gln Asn Trp Arg Tyr Ala Thr Gly Gly Trp Glu
                245                 250                 255

Thr Val Phe Arg Pro Val Ser Glu Thr Cys Thr Asp Arg Ser Gly Ile
            260                 265                 270

Ser Thr Gly His Trp Ser Gly Glu Val Lys Asp Lys Asn Val Gln Val
        275                 280                 285

Val Glu Leu Pro Ile Val Asp Ser Leu His Pro Arg Pro Pro Tyr Leu
    290                 295                 300

Pro Leu Ala Val Pro Glu Asp Leu Ala Asp Arg Leu Val Arg Val His
305                 310                 315                 320

Gly Asp Pro Ala Val Trp Trp Val Ser Gln Phe Val Lys Tyr Leu Ile
                325                 330                 335

Arg Pro Gln Pro Trp Leu Glu Lys Glu Ile Glu Glu Ala Thr Lys Lys
            340                 345                 350

Leu Gly Phe Lys His Pro Val Ile Gly Val His Val Arg Arg Thr Asp
        355                 360                 365

Lys Val Gly Thr Glu Ala Ala Phe His Pro Ile Glu Glu Tyr Met Val
    370                 375                 380

His Val Glu Glu His Phe Gln Leu Leu Ala Arg Arg Met Gln Val Asp
385                 390                 395                 400

Lys Lys Arg Val Tyr Leu Ala Thr Asp Asp Pro Ser Leu Leu Lys Glu
                405                 410                 415

Ala Lys Thr Lys Tyr Pro Asn Tyr Glu Phe Ile Ser Asp Asn Ser Ile
            420                 425                 430

Ser Trp Ser Ala Gly Leu His Asn Arg Tyr Thr Glu Asn Ser Leu Arg
        435                 440                 445

Gly Val Ile Leu Asp Ile His Phe Leu Ser Gln Ala Asp Phe Leu Val
    450                 455                 460

Cys Thr Phe Ser Ser Gln Val Cys Arg Val Ala Tyr Glu Ile Met Gln
465                 470                 475                 480

Thr Leu His Pro Asp Ala Ser Ala Asn Phe His Ser Leu Asp Asp Ile
                485                 490                 495

Tyr Tyr Phe Gly Gly Gln Asn Ala His Asn Gln Ile Ala Ile Tyr Ala
            500                 505                 510

His Gln Pro Arg Thr Ala Asp Glu Ile Pro Met Glu Pro Gly Asp Ile
        515                 520                 525

Ile Gly Val Ala Gly Asn His Trp Asp Gly Tyr Ser Lys Gly Val Asn
    530                 535                 540

Arg Lys Leu Gly Arg Thr Gly Leu Tyr Pro Ser Tyr Lys Val Arg Glu
545                 550                 555                 560

Lys Ile Glu Thr Val Lys Tyr Pro Thr Tyr Pro Glu Ala Glu Lys
                565                 570                 575

<210> 3
<211> 20
<212> DNA
<213> Artificial Sequence
<220>
<223> Description of Artificial Sequence: primer

<400> 3
tggttcctgg cgttggatta                                                20

<210> 4
<211> 20
<212> DNA
<213> Artificial Sequence
<220>
<223> Description of Artificial Sequence: primer

<400> 4
ggatatgtgg ggtacttgac                                                    20

<210> 5
<211> 20
<212> DNA
<213> Artificial Sequence
<220>
<223> Description of Artificial Sequence: primer
<400> 5
cgtcttcaaa gcaagtggat                                                    20

## Description

### TECHNICAL FIELD

The present invention relates to a plant cell having an animal-type sugar chain adding function, a plant regenerated from the plant cell, a method for producing the plant cell, and a method for producing a glycoproteins having an animal-type sugar chain using the plant cell.

### BACKGROUND ART

Apart from conventional and classical breeding methods, plant cells can be modified with genetic engineering technology, with the advent of which otherwise infeasible or useful traits can be conferred to plant cells. To date, for example, plants having disease-resistant, herbicide-resistant, long-lasting properties and the like have been created and utilized. Recently, useful proteins, which are conventionally produced by animal cells, yeast, E. coli, and the like, have been produced by plant cells or plants.

Examples of simple proteins and glycoproteins expressed by plant cells or plants which have been reported up until the present time include the following.

For α-1-antitrypsin, Appl Microbiol Biotechnol 1999 Oct: 52(4):51,6-23 Terashima M, Murai Y, Kawamura M, Nakanishi S, Stoltz T, Chen L, Drohan W, Rodriguez RL, Katoh S; for α-amylase, Biotechnology (NY) 1992 Mar; 10(3):292-6 Pen J, Molendijk L, Quax WJ, Sijmons PC, van Ooyen AJ, van den Elzen PJ, Rietveld K, Hoekema A; for hemoglobin, Nature 1997 Mar 6; 386(6620):29-30 Dieryck W, Pagnier J, Poyart C, Marden MC, Gruber V, BouRNAt P, Baudino S, Merot B; forxylanase, Nat Biotechnol 1999 May; 17(5):466-9 "Production of recombinant proteins in plant root exudates". Borisjuk NV, Borisjuk LG. Logendra S, Petersen F, Gleba Y, Raskin I: for antibodies, Eur J Biochem 1999 Jun; 262(3):810-6 Fischer R, Schumann D, Zimmermann S, Drossard J, Sack M, Schillberg S, J Immunol Methods 1999 Jun 24; 226(1-2):1-10 Fischer R, Liao YC, Drossard J. Curr Top Microbiol Immunol 1999; 236:275-92 Ma JK, Vine ND, J Immunol Methods 1998 Nov 1; 220(1-2):69-75 Vetch T, Yusibov V, Koprowski H; for phytase, Biochem Biophys Res Commun 1999 Oct 14; 264(1) :201-6 "Characterization of recombinant fungal phytase (phyA) expressed in tobacco leaves". Mullah AH, Sethumadhavan K, MullaneyEJ, Ziegelhoffer T, Austin-Phillips S, Plant Physiol 1997 Jul: 114(3):1103-11 "Secretion of active recombinant phytase from soybean cell-suspension cultures". Li J, Hegeman CE, Hanlon RW, Lacy GH, Denbow MD, Grabau EA: for human serum albumin, Biotechnology (NY) 1990 Mar; 8(3):217-21 "Production of correctly processed human serum albumin in transgenic plants" . Sijmons PC, Dekker BM, Schrammeijer B, Verwoerd TC, van den Elzen PJ, Hoekema A; for human lactalbumin, J Biochem (Tokyo) 1998 Mar; 123(3) : 440-4 "Expression of human alpha-lactalbumin in transgenic tobacco". Takase K, Hagiwara K; for human interferon, J Interferon Res 1992 Dec; 12(6):449-53 Edelbaum O, Stein D, Holland N, Gafni Y, Livneh O, Novick D, Rubinstein M, Sela I; for human iduronidase, Curr Top Microbiol Immunol 1999; 240:95-118 "Transgenic plants for therapeutic proteins: linking upstream and downstream strategies". Cramer CL, Boothe JG, Oishi KK; for GM-CSF, CMAJ 1995 Aug 15; 153(4) : 427-9 Robinson A; for hirudin, Plant Mol Biol 1995 Dec; 29(6):1167-80 "Production of biologically active hirudin in plant seeds using oleosin partitioning". Parmenter DL, Boothe JG, van Rooijen GJ, Yeung EC, Moloney MM; for human lactoferrin, Protein Expr Purif 1998 Jun; 13(1):127-35 "Production of human lactoferrin in transgenic tobacco plants". Salmon V, Legrand D, Slomianny MC, el Yazidi I, Spik G, Gruber V, BouRNAt P, Olagnier B, Mison D, Theisen M, Merot B Plant Physiol 1994 Nov; 106(3) : 977-81 "Expression of a human lactoferrin cDNA in tobacco cells produces antibacterial protein(s)". Mitra A, Zhang Z; for inhibitor peptides of angiotensin transferase (tomato and tobacco), Biotechnology (NY) 1993 Aug; 11(8):930-2 Hamamoto H, Sugiyama Y, Nakagawa N, Hashida E, Matsunaga Y, Takemoto S, Watanabe Y, Okada Y; for polyhydroxybutylene, Nat Biotechnol 1999 Oct; 17(10):1011-6 "Metabolic engineering of Arabidopsis and Brassica for poly(3-hydroxybutyrate-co-3-hydroxyvalerate) copolymer production". Slater S, Mitsky TA, Houmiel KL, Hao M, Reiser SE, Taylor NB. Tran M, Valentin HE, Rodriguez DJ, Stone DA, Padgette SR, Kishore G, Gruys KJ Planta 1999 Oct; 209(4):547-50 "Poly(beta-hydroxybutyrate) production in oilseed leukoplasts of brassica napus". Houmiel KL, Slater S, Broyles D, Casagrande L, Colburn S, Gonzalez K, Mitsky TA, Reiser SE. Shah D, Taylor NB, Tran M, Valentin HE, Gruys KJ; for glucocerebrosidase, Ann NY Acad Sci 1996 May 25; 792:62-71 "Bioproduction of human enzymes in transgenic tobacco". Cramer CL, Weissenborn DL, Oishi KK, Grabau EA, Bennett S, Ponce E, Grabowski GA, Radin DN Curr Top Microbiol Immunol 1999; 240:95-118 "Transgenic plants for therapeutic proteins: linking upstream and downstream strategies". Cramer CL, Boothe JG, Oishi KK; for glucuronidase, Adv Exp Med Biol 1999; 464:127-47 "Molecular farming of industrial proteins from transgenic maize". Hood EE, Kusnadi A, Nikolov Z, Howard JA Biotechnol Bioeng 1998 Oct 5; 60(1):44-52, "Processing of transgenic corn seed and its effect on the recovery of recombinant beta-glucuronidase". Kusnadi AR, Evangelista RL, Hood EE, Howard JA, Nikolov ZL; for erythropoietin, Plant Mol Biol 1995 Mar; 27(6):1163-72 Matsumoto S, Ikura K, Ueda M, Sasaki R Biosci Biotechnol Biochem 1993 Aug; 57(8):1249-52 Matsumoto S, Ishii A, Ikura K, Ueda M, Sasaki R glutamic acid decarboxylase Nat Med 1997 Jul; 3(7):793-6 Ma SW, Zhao DL, Yin ZQ, Mukherjee R, Singh B, Qin HY, Stiller CR, Jevnikar AM Adv Exp Med Biol 1999; 464:179-94 Ma S, Jevnikar AM, or the like.

The advantage of using plant cells or plants for the production of useful proteins is that plant cells and plants are capable of adding a sugar chain to a protein.

E. coli generally used for the production of recombinant proteins does not have a sugar chain adding function. Yeast has a sugar chain adding function, but adds a sugar chain having a structure different from that of animals. In animals, the structure of an added sugar chain varies among species. Even in the same animal entity, it has been found that the structure of an added sugar chain varies largely depending on tissue, stages in development and differentiation, or the like.

In general, the sugar chain structure of a glycoprotein is classified into two categories according to the way in which the sugar chain is linked to the glycoprotein. One type of sugar chain is an N-linked sugar chain which is linked to an asparagine residue of a protein. The other is an O-linked sugar chain which is linked to serine or threonine residues of a protein. As for N-linked sugar chains, there are high mannose type sugar chains, complex type sugar chains, and hybrid type sugar chains in animals, plants, insects, yeast, and the like.

A glycoprotein sugar chain has a core structure (core sugar chain). A core sugar chain is first synthesized in the form of a complex with lipid in an endoplasmic reticulum of a cell, and then transferred to a protein (Annu Rev Biochem 1985; 54:631-64 Kornfeld R, Kornfeld S). Thereafter, the protein having the transferred core sugar chain is transported from the endoplasmic reticulum to a Golgi body in which sugars are further added to the core sugar chain so that the chain is elongated. The sugar chain elongation in a Golgi body is called terminal sugar chain synthesis, which varies considerably among species.

Further, fucose residues are linked to N-acetylglucosamine residues in the reducing terminal portion of the core sugar chain in various manners, which depend on the species concerned (Biochim Biophys Acta 1999 Dec 6; 1473(1):21,6-36 Staudacher E, Altmann F, Wilson IB, Marz L).

As described above, plants have a sugar chain adding mechanism as animals do. Plants are potential hosts for the production of useful glycoproteins. However, even though the produced proteins are intended to have physiological activity, some such proteins do not exhibit an inherent activity as physiologically active proteins if the proteins are not successfully modified after translation (particularly by addition of a sugar chain). Further, plants have a sugar chain addition mechanism different from that of animals, particularly that of humans. Therefore, a sugar chain having a structure different from that of an intended animal-type sugar chain may be added to the protein, and the resultant protein is likely to be antigenic to a human (Glycobiology 1999 Apr; 9 (4): 365-72 Cabanes-Macheteau M, Fitchette-Laine AC, Loutelier-Bourhis C, Lange C, Vine ND, Ma JK, Lerouge P, Faye L).

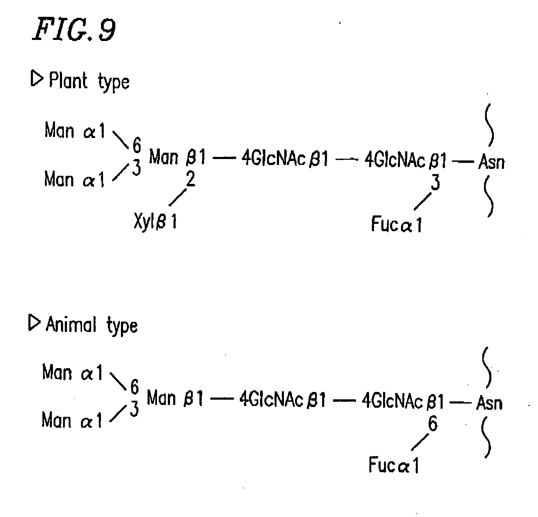

A characteristic structure of a plant sugar chain is the way in which a fucose residue is linked to an N-acetylglucosamine residue existing in a reducing terminal portion of a core sugar chain. It has been reported that such a linkage varies among species (Biochim Biophys Acta 1999 Dec 6; 1473(1):21 6-36 Staudacher E, Altmann F, Wilson IB, Marz L). For plants, an α1,3-linkage has been reported (Biosci Biotechnol Biochem 1999 Jan; 63(1):35-9 Palacpac NQ, Kimura Y, Fujiyama K, Yoshida T, Seki T; Biosci Biotechnol Hiochem 1997 Nov; 61(11):1866-71 Kimura Y,Ohno A, Takagi S; Eur J Biochem 1991 Jul 1; 199(1):169-79 Sturm A). For mammals such as humans and mice, an α1,6-linkage has been reported (Glycobiology 1991 Sep; 1(4):337-46 Takeuchi M, Kobata A). In Figure 9, complex-type sugar chain structures of a plant and an animal are shown. For insect cells, both α1,3-linkages and α1,6-linkages have been found (Glycoconj J 1998 Nov: 15(11):1055-70 Wilson IB, Altmann F; Eur J Biochem 1991 Aug 1: 199(3):745-51 Staudacher E, Altmann F, Glossl J, Marz L, Schachter H, Kamerling JP, Hard K, Vliegenthart JF).

A sugar chain portion having α1,3 glycoprotein linkages derived from plants and insects is likely to be antigenic to humans (Glycoconj J 1998 Nov; 15(11):1055-70 Wilson IB, Altmann F; Int Arch Allergy Immunol 1999 Feb-Apr; 118(2-4):4113 Petersen A, Grobe K, Schramm G, Vieths S, Altmann F, Schlaak M, Backer WM; Int Arch Allergy Immunol 1999 Sep; 120(1):30-42 Fotisch K, Altmann F, Haustein D, Vieths S).

The gene of an enzyme for adding a fucose residue to an N-acetylglucosamine residue, α1,3-fucosyl transferase cDNA, has been cloned from a plant, a mung bean (J Biol Chem 1999 Jul 30; 274(31):21830-9 Leiter H. Mucha J, Staudacher E, Grimm R, Glossl J, Altmann F). For mammals, α1,6-fucosyl transferase cDNA has been cloned from humans and pigs (J Biochem (Tokyo) 1997 Mar; 121(3):626-32 Yanagidani S, Uozumi N, Ihara Y, Miyoshi E, Yamaguchi N, Taniguchi N; J Biol Chem 1996 Nov 1; 271(44):27810-7 Uozumi N, Yanagidani s, Miyoshi E, Ihara Y, Sakuma T, Gao CX, Teshima T, Fujii S, Shiba T, Taniguchi N).

A variant of an N-acetylglucosaminyl transferase I gene has been obtained from Arabidopsis thaliana. In this variant, sugar chain processing is arrested after N-acetylglucosaminyl transferase I (Plant Physiol 1993 Aug: 102(4):1109-18 von Schaewen A, Sturm A, O'Neill J, Chrispeels MJ). When N-acetylglucosaminyl transferase I cDNA derived from a human was introduced into the variant, N-acetylglucosaminyl transferase activity was recovered (Proc Natl Acad Sci USA 1994 Mar 1: 91(5):1829-33 Gomez L, Chrispeels MJ). Conversely, when N-acetylglucosaminyl transferase I cDNA derived from Arabidopsis thaliana was introduced into the CHO cell variant Lecl which has no N-acetylglucosaminyl transferase activity, the N-acetylglucosaminyl transferase activity of the CHO cells was recovered (Biochem Biophys Res Commun 1999 Aug 11; 261(3):829-32 Bakker H, Lommen A, Jordi W, Stiekema W, Bosch D).

Further, it has been found that out of genes relevant to the biosynthesis of a nod factor in a nitrogen-fixing bacterium Rhizobium sp. NGR234, a nodZ gene encodes fucose transferase (J Bacteriol 1997 Aug; 179(16):5087-93 Quesada-Vincens D, Fellay R, Nasim T, Viprey V, Burger U, Prome JC, Broughton WJ, Jabbouri S).

Olsthoorn et al. have shown that in Mesorhizobium loti NZP2213, α1,3-fucosyl transferase is involved in the nod factor biosynthesis (Biochemistry 1998 Jun 23:37(25):9024-32 Olsthoorn MMA, Lopez-Lara IM, Petersen BO, Bock K, Haverkamp J, Spaink HP, Thomas-Oates JE). A NodZ protein derived from Mesorhizobium loti transfers the fucose residue of GDP-β-fucose to the C6 position of the reducing terminal N-acetylglucosamine residue of a chitin oligosaccharide (Proc Natl Acad Sci USA 1997 Apr 29; 94(9):4336-41 Quinto C, Wijfjes AHM, Bloemberg GV, Blok-Tip L, Lopez-Lara IM, Lugtenberg BJ, Thomas-Oates JE, Spaink HP). This NodZ protein has the same enzyme activity as that of α1,6-fucosyl transferase derived from an animal, but has substantially no homology with it at the amino acid sequence level (Glycobiology 1991 Dec: 1(6):577-84 Macher BA, Holmes EH, Swiedler SJ, Stults CL, Srnka CA: Histochem J 1992 Nov; 24(11):761-70 de Vries T, van den Eijnden DH).

Further, when a NodZ protein derived from M. loti having α1,6-fucosyl transferase activity was microinjected to a fertilized egg of zebrafish, deformation occurs in the embryogenesis of a body and a caudal fin (Proo Natl Acad Sci USA 1997 Jul 22: 94(15):7982-6 Bakkers J, Semino CE, Stroband H, Kijne JW, Robbins PW, Spaink HP: Ann N Y Acad Sci 1998 Apr 15; 842:49-54 Semino CE, Allende ML, Bakkers J, Spaink HP, Robbins PP).

When β1,4-galactose transferase gene cDNA derived from a human was introduced to a cultured tobacco cell, a sugar chain structure having a transf erred galactose residue was obtained in the plant cell. With the introduction of the human-derived sugar transferase gene, the processing pathway of a sugar chain in a plant cell could be remodeled (Proc Natl Acad Sci USA 1999 Apr 13; 96(8) :4692-7 Palacpac NQ, Yoshida S, Sakai H, Kimura Y, Fujiyama K, Yoshida T, Seki T).

Further, Steinkellner has shown that using N-acetylglucosaminyl transferase I cDNA cloned from tobacco, the expression of N-acetylglucosaminyl transferase gene could be suppressed, or the expression amount could be reduced, by an antisense gene suppressing method or a post-transcription gene silencing method (International Molecular Farming Conference, London, Ontario, Canada, Aug. 29 Sept.1, 1999, Abstract Book, W79, p. 46, Steinkellner H).

The inventors have diligently studied the above-described problems caused by the existence of different sugar chain adding functions in different organisms and completed the present invention. The present invention is provided to solve the above-described conventional problems by introducing the fucose transferase gene, which does not originally exist in plant cells, into a plant cell. The objective of the present invention is to provide a plant cell having an animal-type sugar chain adding function, a plant regenerated from the plant cell, a method for producing the plant cell, and a method for producing an animal type glycoprotein using the plant cell.

### DISCLOSURE OF THE INVENTION

The present invention relates to a plant cell having an animal-type sugar chain adding function. The plant cell has an introduced gene encoding an enzyme derived from an animal, and the enzyme can transfer a fucose residue to a reducing terminal acetylglucosamine residue of a sugar chain of a glycoprotein.

Preferably, the enzyme derived from an animal is α1,6-fucosyl transferase.

In one aspect, the present invention relates a plant regenerated from the plant cell.

In another aspect, the present invention relates to a method for producing a plant cell having an animal-type sugar chain adding function. The method comprises the step of introducing into the plant cell a gene encoding an enzyme derived from an animal, in which the enzyme can transfer a fucose residue to a reducing terminal acetylglucosamine residue of a sugar chain of a glycoprotein.

In yet another aspect, the present invention relates to a method for producing a glycoprotein having an animal-type sugar chain. The method comprises the step of transforming a plant cell by introducing into the plant cell a gene encoding an enzyme derived from an animal and a gene encoding an exogenous glycoprotein, in which the enzyme can transfer a fucose residue to a reducing terminal acetylglucosamine residue of a glycoprotein, and cultivating the resultant transformed plant cell.

The present invention also relates to a glycoprotein produced by the above method. This glycoprotein has an animal-type sugar chain.

### BRIEF DESCRIPTION OF THE DRAWINGS

Figure 1 is a diagram showing the construction of the vector pBI221-FT used in the production of a plant cell of the present invention. The unique SacI site in the pBI221-FT vector was converted to a Sall site. An α1,6-FT gene was inserted to the site.
Figure 2 is a diagram showing the construction of the vector pGPTV-HPT-FT used in production of a plant cell of the present invention.
Figure 3 is a photograph of a color-developed gel after electrophoresis for genomic DNA which was prepares from transformants BY2-FT 2 to 13 and amplified by PCR.
Figure 4 is a photograph of a color-developed gel after electrophoresis for genomic DNA which was obtained by amplifying RNA prepared from transformants BY2-FT 2, 3, 4 and 6 by RT-PCR.
Figure 5 is a diagram showing a result of HPLC analysis.
Figure 6 is a diagram showing a result of HPLC analysis.
Figure 7 is a diagram showing a result of HPLC analysis.
Figure 8 is a photograph of a color-developed PVDF membrane after blotting a gel electrophoresis gel, showing a result of analysis of a glycoprotein produced by a plant cell of the present invention using lectin.
Figure 9 is a diagram showing complex-type sugar chain structures of a plant and an animal. In the core portion of a complex-type sugar chain structure, a plant-type sugar chain includes a xylose residue, while an animal-type sugar chain includes a xylose residue. Further, a fucose residue α1,6-linked to the most inner N-acetylglucosamine in an animal-type sugar chain, while a fucose residue α1,3-linked to the most inner N-acetylglucosamine in a plant-type sugar chain.
Figure 10 is a schematic diagram showing a structure of a substrate sugar chain used in measurement of α1,6-FT, and an activity measurement system.
Figure 11 is a chromatogram of HPLC analysis of a glycoprotein produced by a transformant BY2-FT3 cultured cell.
Figure 12 is a diagram showing the results of analysis of a sugar chain structure (high mannose-type sugar chain) of a glycoprotein produced by a transformant BY2-FT3 cultured cell.
Figure 13 is a diagram showing the results of analysis of the sugar chain structure (complex-type sugar chain) of a glycoprotein produced by a transformant BY2-FT3 cultured cell.
Figure 14 is a diagram showing the results of analysis of the sugar chain structure (α1,6-fucose-linked sugar chain) of a glycoprotein produced by a transformant BY2-FT3 cultured cell.
Figure 15 is a photograph of a color-developed gel after electrophoresis of genomic DNA amplified by PCR which was prepared from transformed plants FT(1), FT(2), FT1, FT2, and FG3.

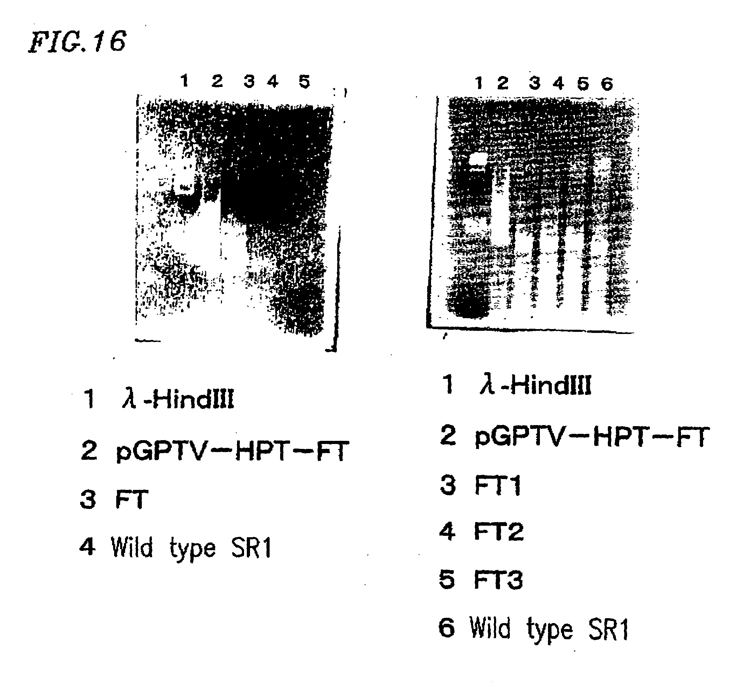

Figure 16 is a photograph of a color-developed PVDF membrane after blotting a gel electrophoresis gel, showing the results of analysis of a glycoprotein produced by a plant cell of the present invention using lectin.

### BEST MODE FOR CARRYING OUT THE INVENTION

Hereinafter, the present invention will be described in detail.

Methods for isolating and analyzing proteins, and immunoassays, which are known in the art, may be used in the present invention, unless otherwise mentioned. These techniques may be performed using commercially available kits, antibodies, labeled materials, and the like. The techniques used in the present invention will be described in the Materials and Methods section below.

A method according to the present invention is directed to a plant cell having an animal-type sugar chain adding function. The term "animal-type sugar chain" as used herein refers to a sugar chain in which a fucose residue is α1,6 linked to an N-acetylglucosamine residue existing at a reducing terminal portion in the core sugar chain of a glycoprotein. Preferably, the fucose residue is linked to an N-acetylglucosamine residue existing in the most core portion of the core sugar chain which is linked to an asparagine residue of a protein.

The plant cells can be any plant cells. The plant cells can be cultured cells, cultured tissue, cultured organs, or a plant. Preferably, the plant cells should be cultured cells, cultured tissue, or cultured organs, and most preferably cultured cells. The type of plant used in the production method of the present invention can be any type of plant that can be used in gene introduction. Examples of types of plants that can be used in the manufacturing method of the present invention include plants in the families of Solanaceae, Poaeae, Brassicaceae, Rosaceae, Leguminosae, Curcurbitaceae, Lamiaceae, Liliaceae, Chenopodiaceae and Umbelliferae.

Examples of plants in the Solanaceae family include plants in the Nicotiana, Solanum, Datura, Lycopersicon and Petunia genera. Specific examples include tobacco, eggplant, potato, tomato, chili pepper, and petunia.

Examples of plants in the Poaeae family include plant s in the Oryza, Hordenum, Secale, Saccharum, Echinochloa and Zea genera. Specific examples include rice, barley, rye, Echinochloa crus-galli, sorghum, and maize.

Examples of plants in the Brassicaceae family include plants in the Raphanus, Brassica, Arabidopsis, Wasabia, and Capsella genera. Specific examples include Japanese white radish, rapeseed, arabidopsis thaliana, Japanese horseradish, and Capsella bursa-pastoris.

Examples of plants in the Rosaceae family include plants in the Orunus, Malus, Pynus, Fragaria, and Rosa genera. Specific examples include plum, peach, apple, pear, Dutch strawberry, and rose.

Examples of plants in the Leguminosae family include plants in the Glycine, Vigna, Phaseolus, Pisum, Vicia, Arachis, Trifolium, Alfalfa, and Medicago genera. Specific examples include soybean, adzuki bean, kidney beans, peas, fava beans, peanuts, clover, and alfalfa.

Examples of plants in the Curcurbitaceae family include plants in the Luffa, Curcurbita, and Cucumis genera. Specific examples include gourd, pumpkin, cucumber, and melon.

Examples of plants in the Lamiaceae family include plants in the Lavandula, Mentha, and Perilla genera. Specific examples include lavender, peppermint, and beefsteak plant.

Examples of plants in the Liliaceae family include plants in the Allium, Lilium, and Tulipa genera. Specific examples include onion, garlic, lily, and tulip.

Examples of plants in the Chenopodiaceae family include plants in the Spinacia genera. A specific example is spinach.

Examples of plants in the Umbelliferae family include plants in the Angelica, Daucus, Cryptotaenia, and Apitum genera. Specific examples include Japanese udo, carrot, honewort, and celery.

Preferably, the plants used in the production method of the present invention should be tobacco, tomato, potato, rice, maize, radish, soybean, peas, alfalfa or spinach. More preferably, the plants used in the production method of the present invention should be tobacco, tomato, potato, maize or soybean.

The "enzyme capable of transferring a fucose residue to a reducing terminal acetylglucosamine residue" refers to an enzyme capable of transferring a fucose residue to a reducing terminal acetylglucosamine residue during the addition of a sugar chain after the synthesis of the protein portion of a glycoprotein in a plant cell. An example of such an enzyme is α1,6-fucosyl transferase. This enzyme causes fucose to be α1,6 linked to N-acetylglucosamine of the N-linked sugar chain closest to the peptide chain of a glycoprotein, where GDP-fucose is used as a sugar donor. The enzyme can be derived from any animal, preferably from a mammal, and more preferably from a human.

Preferably, this enzyme is an enzyme localized in cell organelles. The inventors believe that the enzyme exists in cell organelles (e.g., endoplasmic reticulum and Golgi body) and causes a fucose residue to be α1,6 linked to N-acetylglucosamine residue existing at a reducing terminal portion of an exogenous protein in a plant cell. Although the inventors do not intend to be constrained to a specific theory.

The "gene of an enzyme capable of transferring a fucose residue to a reducing terminal acetylglucosamine residue" may be a gene isolated from any animal cell using a nucleotide sequence encoding the enzyme, or a commercially available one. These enzymes may be modified to be suited for expression in plants. For such isolation and modification, there are methods known to those skilled in the art.

For example, for mammals, α1,6-fucosyl transferase cDNA has been cloned from a human and a pig (J Biochem (Tokyo) 1997 Mar; 121(3):626-32 Yanagidani S, Uozumi N. Ihara Y, Miyoshi E, Yamaguchi N, Taniguchi N; Japanese Laid-Open Publication No. 10-84975, Japanese Laid-Open Publication No. 10-4959; J Biol Chem 1996 Nov 1; 271(44):27810-7 Uozumi N, Yanagidani S, Miyoshi E, Ihara Y, Sakuma T, Gao CX, Teshima T, Fujii S, Shiba T, Taniguchi N; Japanese Laid-Open Publication No. 10-4969, Japanese Laid-Open Publication No. 9-201191). The structure of the cDNA has been shown.

The term "gene" as used herein refers to the structural gene portion. A control sequence such as a promoter, an operator and a terminator can be linked to the gene so as to properly express the gene in a plant.

The term "exogenous glycoproteins" refers to glycoproteins whose expression in plants is the result of genetic engineering methodologies. Examples of these exogenous glycoproteins include enzymes, hormones, cytokines, antibodies, vaccines, receptors and serum proteins. Examples of enzymes include horseradish peroxidase, kinase, glucocerebrosidase, α-galactosidase, tissue-type plasminogen activator (TPa), and HMG-CoA reductase. Examples of hormones and cytokines include enkephalin, interferon alpha, GM-CSF, G-CSF, chorion stimulating hormone, interleukin-2, interferon-beta, interferon-gamma, erythropoietin, vascular endothelial growth factor, human choriogonadotropin (HCG), leuteinizing hormone (LH), thyroid stimulating hormone (TSH), prolactin, and ovary stimulating hormone. Examples of antibodies include IgG and scFv. Examples of vaccines include antigens such as Hepatitis B surface antigen, rotavirus antigen, Escherichia coli enterotoxin, malaria antigen, rabies virus G protein, and HIV virus glycoprotein (e.g., gp120). Examples of receptors and matrix proteins include EGF receptors, fibronectin, α1-antitrypsin, and coagulation factor VIII. Examples of serum proteins include albumin, complement proteins, plasminogen, corticosteroid-binding globulin, throxine-binding globulin, and protein C.

The term "genes of exogeneous glycoproteins" refers to genes isolated from any animal cell using a nucleotide sequence encoding the gene, or a commercially available one. These genes may be modified to be suitable for expression in plants.

The gene of the enzyme capable of transferring a fucose residue to a reducing terminal N-acetylglucosamine residue and the genes of exogenous glycoproteins can be introduced to plant cells using a method known in the art. These genes can be introduced separately or simultaneously. Examples of methods for introducing genes to plant cells include the Agrobacteriummethod, the electroporation method and the particle bombardment method.

Suitable methods of transforming plant cells include microinjection (Crossway et al., BioTechniques 4:320-334 (1986)), electroporation (Riggs et al., Proc. Natl. Acad. Sci. USA 83:5602-5606 (1986), Agrobaaterium-mediated transformation (Hinchee et al., Biotechnology 6:915-921 (1988): See also, Ishida et al., Nature Biotechnology 14:745-750 (June 1996) for maize transformation), direct gene transfer (Paszkowski et al., EMBO J. 3:2717-2722 (1984); Hayashimoto et al., Plant Physiol 93:857-863 (1990) (rtce)), and ballistic particle acceleration using devices available from Agracetus, Inc., Madison, Wis. and Dupont, Inc., Wilmington, Del. (see, for example, Sanford et al., U.S. Pat. No. 4,945,050; and McCabe et al., Biotechnology 6:923-926 (1988)). See also, Weissinger et al., Annual Rev. Genet. 22:421-477 (1988); Sanford et al., Particulate Science and Technology 5.27-37 91987) (onion): Svab et al., Proc. Natl. Acad. Sci. USA 87:8526-8530 (1990) (tobacco chloroplast); Christou et al., Plant Physiol. 87:671-674 (1988)(soybean); McCabe et al., Bio/Technology 6.923-926 (1988) (soybean); Klein et al., Proc. Natl. Acad. Sci. USA, 85:4305-4309 (1988)(maize); Klein et al., Bio/Technology 6:559-563 (1988) (maize); Klein et al., Plant Physiol. 91:440-444 (1988) (maize): Fromm et al., Bio/Technology 8:833-839 (1990): and Gordon-Kamm et al., Plant Cell 2: 603-618 (1990) (maize); Koziel et al., Biotechnology 11: 194-200 (1993) (maize): Shimamoto et al. , Nature 338 : 274-277 (1989) (rice); Christou et al., Biotechnology 9: 957-962 (1991) (rice); Datta et al. , Biol/Technology 3:736-740 (1990) (rice): European Patent Application EP 0 332 581 (orchardgrass and other Pooideae); Vasil et al., Biotechnology 11: 1553-1558 (1993) (wheat); Weeks et al., Plant Physiol. 102: 1077-1084 (1993) (wheat); Wan et al., Plant Physiol. 104: 37-48 (1994) (barley); Jahne et al., Theor. Appl. Genet. 89:525-533 (1994) (barley): Umbeck et al., Bio/Technology 5: 263-266 (1987) (cotton); Casas et al., Proc. Natl. Acad. Sci. USA 90:11212-11216 (December 1993) (sorghum); Somers et al., Bio/Technology 10:1589-1594 (December 1992) (oat); Torbert et al., Plant Cell Reports 14:635-640 (1995) (oat): Weeks et al., Plant Physiol. 102:1077-1084 (1993) (wheat): Chang et al., WO 94/13822 (wheat) and Nehra et al., The Plant Journal 5:285-297 (1994) (wheat). A particularly preferred set of embodiments for the introduction of recombinant DNA molecules into maize by microprojectile bombardment can be found in Koziel et al., Biotechnology 11: 194-200 (1993), Hill et al., Euphytica 85:119-123 (1995) and Koziel et al., Annals of the New York Academy of Sciences 792:164-171 (1996). An additional preferred embodiment is the protoplast transformation method for maize as disclosed in EP 0 292 435. Transformation of plants can be undertaken with a single DNA species or multiple DNA species (i.e. co-transformation) and both these techniques are suitable for use with the peroxidase coding sequence.

The expression of genes introduced into plant cells can be observed using any method known in the art. Examples of such methods include silver staining or augmentation, Western blotting, Northern hybridization, and enzyme activity detection. Cells that express the introduced genes are referred to as transformed cells.

Transformed cells, which express both the enzyme capable of transferring a fucose residue to a reducing terminal N-acetylglucosamine residue and the exogenous glycoproteins, express exogenous glycoproteins with animal-type sugar chains. In other words, the transformed cells have animal-type sugar chain adding functions. By cultivating these transformed cells, glycoproteins with animal-type sugar chains can be mass produced. Animal-type glycoproteins contain core sugar chains and outside sugar chains. The core sugar chains consist essentially of at least one mannose or one or more acetylglucosamines. The outsides sugar chains in these glycoproteins contain non-reducing terminal sugar chain portions. The outside sugar chains can have a straight chain structure or abranched chain structure. Preferably, the outside sugar chains can have a branched chain structure. The branched sugar chain portion has a mono- , bi-, tri- or tetra structure. The glycoproteins produced by these transformed cells preferably contains any fucose residue which is α1,6 linked to N-acetylglucosamine of the N-linked sugar chain closest to the peptide chain of a glycoprotein.

These transformed plant cells may be held in the form of cultured cells or may be differentiated into specific tissues or organs. Alternatively, they can also be regenerated into plants. In this case, the transformed plant cells can be present in the entire plant or in specific portions of the plant, such as the seed, fruit, nut, leaf, root, stem or flower of the plant.

For the culture, differentiation or regeneration of the transformed plant cell, means and culture mediums known in the art are used. Examples of the medium include Murashige-Skoog (MS) medium, Gamborg B5 (B) medium, White medium and Nitsch & Nitsch (Nitsch) medium, however, the present invention is not limited thereto. These mediums are usually used after adding thereto an appropriate amount of a plant growth control substance (e.g., plant hormone) and the like.

Application of these systems to different plant strains depends upon the ability to regenerate that particular plant strain from protoplasts. Illustrative methods for the regeneration of cereals from protoplasts are described (Fujimura et al. , Plant Tissue Culture Letters, 2:74, 1985: Toriyama et al., Theor. Appl. Genet., 73:16, 1986; Yamada et al., Plant Cell Rep., 4:85, 1986; . Abdullah et al.. Biotechnology, 4:1087, 1986).

To transform plant strains that cannot be successfully regenerated from protoplasts, other ways to introduce DNA into intact cells or tissues can be utilized. For example, regeneration of cereals from immature embryos or explants can be effected as described (Vasil, Biotechnology, 6:397, 1988).

Agrobacterium-mediated transfer is also a widely applicable system for introducing genes into plant cells because the DNA can be introduced into whole plant tissues, thereby bypassing the need for regeneration of an intact plant from a protoplast. The use of Agrobactexxum-mediated plant integrating vectors to introduce DNA into plant cells is well known in the art. See, for example, the methods described above.

Glycoproteins with animal-type sugar chains produced by the transformed plant cells may be isolated or extracted from the plant cells. The method for isolating the glycoproteins can be any method known in the art. The glycoproteins of the present invention can be used in foodstuffs while remaining inside the transformed cells. The glycoproteins produced by the plant cells of the present invention can be administered to animals, particularly a human, without antigenicity because of the added animal-type sugar chains.

### (Examples)

The materials, reagents, and operating procedure used in Examples will all be described in the Materials and Methods section below.

### (Example 1: Introduction of α1,6-fucosyl transferase gene (hereinafter referred to as α1,6-FT) into cultured tobacco cells)

Introduction of the α1,6-fucosyl transferase gene into cultured tobacco cells was conducted using Agrobacterium capable of infecting plant cells. A. tumefaciens is often used to transform dicotyledons. Recently, it has been shown that a group of genes encoded in the vir region on a Ti plasmid are involved in oncogenesis. When infecting plants, Agrobacterium receives phenol substance secreted by dicotyledons as an infection signal, and then activates the transcription of the vir gene group. As a result, several proteins encoded by the vir genes cut, transfer, and incorporate a T-DNA gene. T-DNA and the vir genes are not individually capable of oncogenesis. Even if T-DNA and the vir genes are present on separate replicons but in the same Agrobacterium cell, T-DNA and the vir genes are collectively capable of oncogenesis. A method for introducing an exogenous gene using a binary vector employs this property.

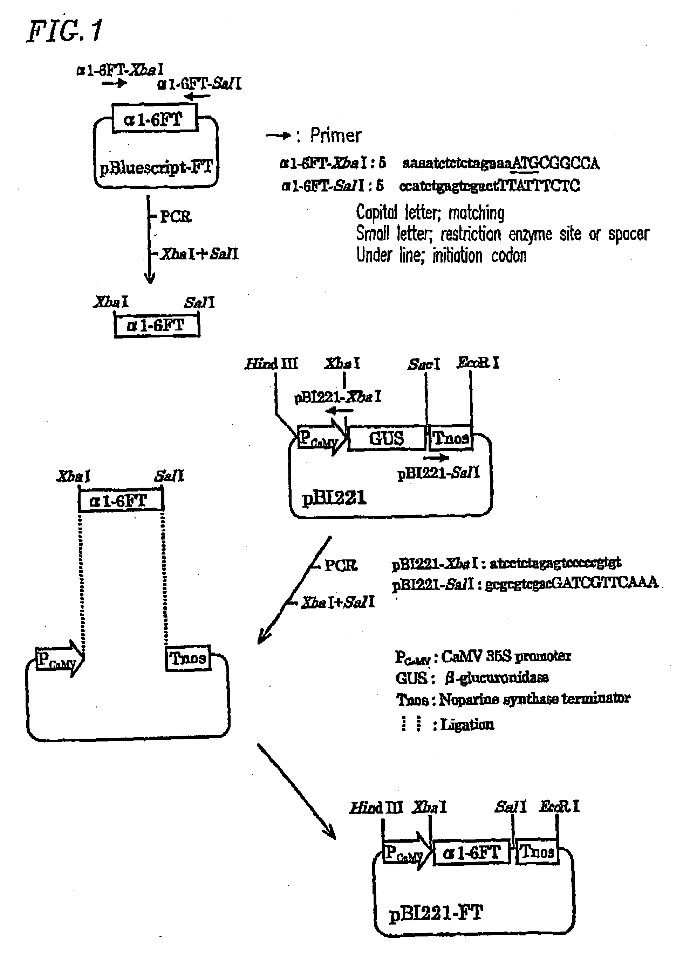

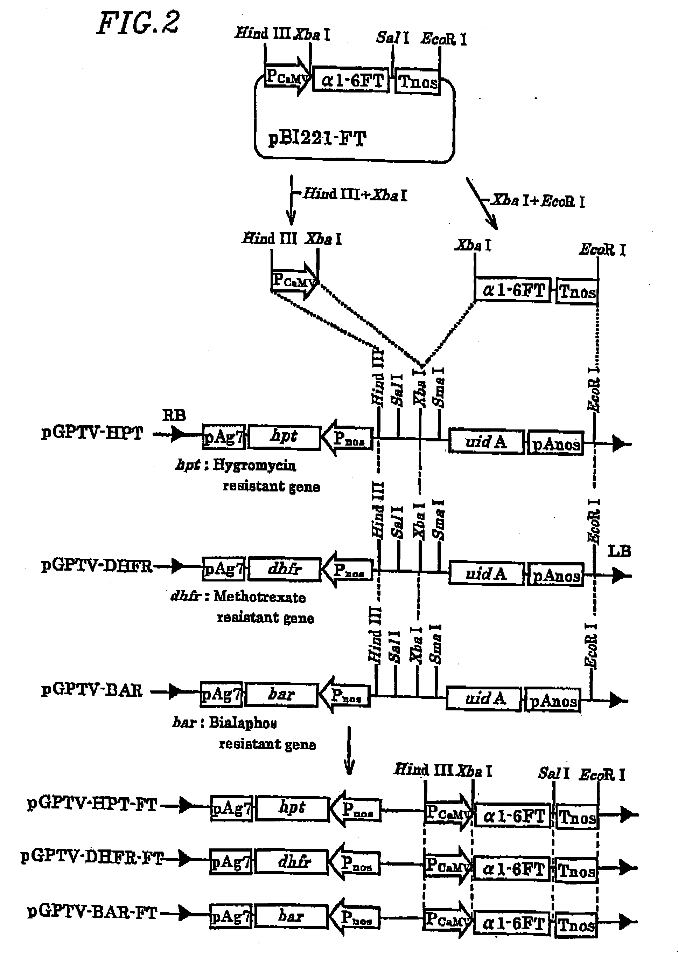

In this example, cDNA (SEQ ID No. 1) of a human-derived α1,6-FT (SEQ ID NO. 2), i.e., sugartransferase (pBluescript-FT obtained by subcloning the α1,6-FT gene was provided by Prof. Naoyuki Taniguchi of the faculty of Medicine of Osaka university) was inserted into a T-DNA region to construct binary vectors, pGPTV-HPT-FT, pGPTV-DHFR-FT, and pGPTV-BAR-FT. A construction scheme of these binary vectors is shown in Figures 1 and 2.

Initially, an α1,6-FT gene fragment amplified by PCR using pBluescript-FT as a template was digested by a restriction enzyme. Similarly, the gene fragment was inserted into the pBI221 vector (CLONTECH Laboratries, Inc.) whose restriction site was modified by PCR to produce a pBI221-FT vector (Figure 1). Primers were produced with reference to a report by Yanagidani et al. (J Biochem (Tokyo) 1997 Mar; 121(3):626-32 Yanagidani S, Uozumi N, Ihara Y, Miyoshi E, Yamaguchi N, Taniguchi N; J Biol chem 1996 Nov 1; 271(44)).

Further, an XbaI-EcoRI fragment including a califlower mosaic virus 35S promotor gene, α1,6-FT, and a nopaline syntase terminator gene was cut out from the pBI221-FT vector. The XbaI-EcoRI fragments were incorporated into three plant transforming binary vector pGPTV-HPT (i.e. , ATCC77389 obtained from ATGC (America Type Culture Collection (12301 Parklawn Drive, Rockbill, Maryland USA 20852), pGPTV-DHFR (i.e., ATCC77390 obtained from ATCC), pGPTV-BAR (i.e., ATCC77391 obtained from ATCC) (Figure 2). These three binary vectors have different drug-resistant genes in theT-DNAregions thereof, thereby making it possible to screen transformed plant cells using different drugs.

The reason the three different drug-resistant expression genes (i.e., pGPTV-HPT-FT, pGPTV-DHFR-FT, and pGPTV-BAR-FT) were prepared is that the drugs used for the screening of the transformed cells, and the introduced sugar transferase have unknown influence on the cells. A drugwhich can certainly be used for screening in this case had not been known. Therefore, three vectors for the expression of α1,6-FT were constructed in advance. The construction of the three vectors was preferable from the view point that the vectors having screening markers with different action mechanisms would be useful when a plurality of exogenous genes are introduced into the same clone in the future.

Of the expression vectors prepared, pGPTV-HPT-FT was used to transform a tobacco BY2 cultured cell in this example.

Agrobacterium was transformed by a Bevan et al. 's triparental mating method (Bevan M. , Nucleic Acid Res., 12, 8711, 1984). Escherichia coli DH5α (suE44, ΔlacU169, (φ801acZΔM15), hsdR17) (Bethesda Research Laboratories Inc.: Focus 8(2), 9 (1986)) having a pGPTV-type plasmid (Plant Mol Biol 1992 Dec; 20(6):1195-7 Hecker D, Kemper E, Schell J, Masterson R), and Escherichia coli HB101 having a helper plasmid pRK2013 (Bevan M., Nucleic Acid Res., 12, 8711, 1984) were cultivated in respective 2xYT media including 12.5 mg/l tetracycline and 50 mg/l kanamycin at 37° C overnight. Agrobacterium tumefaciens EHA101 (Elizanbeth E.H., J. Bacteriol., 168, 1291, 1986) was cultivated in 2×YT medium including 50 mg/l kanamycin and 25 mg/l chloramphenicol at 28° C for two nights. Then, 1.5 ml of each cultured medium was removed and placed into an Eppendorf tube. After the cells of each strain were collected, the cells were rinsed three times with LB medium. The cells obtained in this manner were then suspended in 100 µl of 2xYT medium, mixed with three types of bacteria, applied to 2xYT agar medium, and cultivated at 28° C, whereby the pGPTV-type plasmids then underwent conjugational transfer from the E. coli to the Agrobacterium. Two days later some of the colonies appearing on the 2 x YT agar plate were removed using a platinum loop, and applied to an LB agar plate containing 50 mg/l kanamycin, 12.5 mg/l tetracycline, and 25 mg/l chloramphenicol. After cultivating the contents for two days at 28° C, a single colony was selected.

Transformation of the cultivated tobacco cells was performed using the method described in An G., Plant Mol. Bio. Manual, A3, 1. First, 100 µl of Agrobacterium EHA101 with a pGPTV-type plasmid cultivated for 48 hours at 28°C in LB medium containing 12.5 mg/l tetracycline, and 4 ml of a suspension of cultivated tobacco cells Nicotiana tabacum L. cv. bright yellow 2 (Strain No. BY2 obtained using Catalog No. RPC1 from the Plant Cell Development Group of the Gene Bank at the Life Science Tsukuba Research Center), in their fourth day of cultivation, were mixed together thoroughly in a petri dish and allowed to stand in a dark place at 25° C. Two days later, some of the solution was removed from the petri dish and the supernatant was separated out using a centrifuge (1000 rpm, 5 minutes). The cell pellet was introduced to a new medium and centrifuged again. The cells were inoculated onto a modified LS agar plate with 20 mg/l, hygromycin and 250 mg/l carbenicillin. This was allowed to stand in darkness at 25°C. After two to three weeks, the cells grown to the callus stage were transferred to a new plate and growing clones were selected. After further two to three weeks, the clones were transferred to 30 ml of a modified LS medium with hygromycin and carbenicillin followed by subcuturing. Since hygromycin was used in the screening, it took a period of time (about 5 weeks) which is about two times as much as usual to obtain a transformed callus. For the resultant transformed callus, screening was repeated in about one month using selection media including hygromycin. Twelve resistant strains were randomly selected from the resistant strains obtained in this manner (BY2-FT 2 to 13) to be used in analysis at a DNA level.

### (Analysis at the DNA Level of BY2-FT cells)

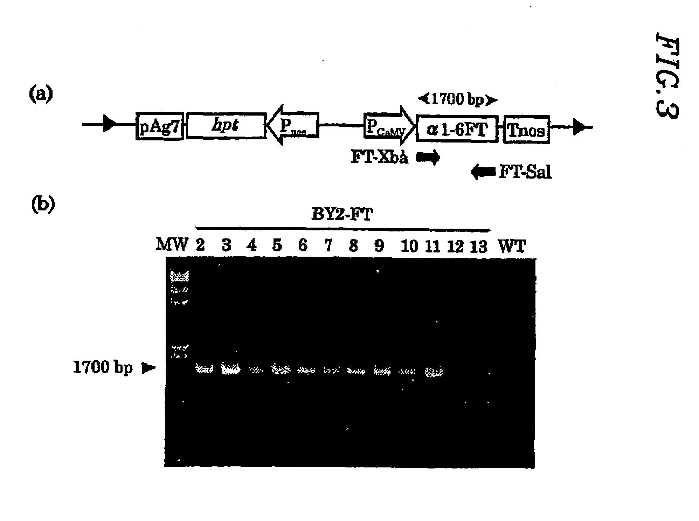

The obtained transformant strains BY2-FT 2 to 13 were studied as follows. Using calluses thereof, genomic DNA was prepared in accordance with a method described in the Materials and Methods section 10 below, and the incorporation of the α1,6-FT gene was examined by PCR (see the Materials and Methods section 12). For the PCR, the following primers were employed: FT-Xba: 5'-TGGTTCCTGGCGTTGGATTA (SEQ ID NO. 3), and FT-Sal: 5'-GGATATGTGGGGTACTTGAC (SEQ ID NO. 4). The obtained PCR amplified products were subjected to electrophoresis in accordance with a method described in the Materials and Methods section 8. The result is shown in Figure 3.

As shown in Figure 3, eleven strains, i.e., BY2-FT 2, 3, 4, 5, 6, 7, 8, 9, 10, 11, and 13 exhibited bands around 1700 bp which were considered to be of the amplified fragments of the α1,6-FT gene region. In contrast, when genomic DNA prepared from a wild-type cultured tobacco cell was used as a template, no band was found (a lane indicated by WT in the right end of Figure 3). Therefore, it was confirmed that the α1,4-FT gene was incorporated in a chromosome of BY2-FT cell.

### (Analysis at the RNA Level of BY2-FT cells)

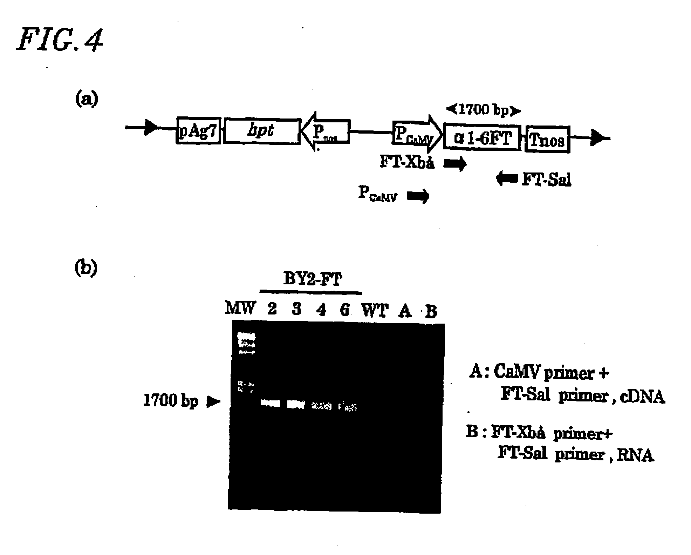

Of the transformants for which the introduction of the α1,6-FT gene was confirmed as a result of the genomic DNA analysis by PCR, four strains BY2-FT 2, 3, 4, and 6 having a high growth rate were studied. RNA thereof was prepared in accordance with a method described in the Materials and Methods section 11 below, and subjected to RT-PCR (see the Materials and Methods section 13 below). The result is shown in Figure 4. RT-PCR was conducted using the same primers as described above, and the resultant amplified products were subjected to electrophoresis under the same conditions as described above in the DNA analysis. As a result, as shown in Figure 4, it was observed that all the four strains exhibited bands around 1700 bp which were considered to be of the amplified fragment of the α1, 6-FT gene. No band was found for a wild-type BY2 specimen (a lane indicated by WT in Figure **4****).** Further, RT-PCR was conducted using a primer designed based on the sequence of a CaMV 35S promoter (CaMV primer) (SEQ ID NO. 5) and a FT-Sal primer, resulting in no band (lane A in Figure 4): CaMV primer: 5'-CGTCTTCAAAGCAAGTGGAT(SEQ ID NO. 5).

In these experiments, there was the possibility that RNA liquid recovered by the kit for preparing RNA specimens was contaminated with genomic DNA. The recovered RNA specimens were all treated with DNase before RT-PCR. In the case of PCR using the RNA specimens after the DNase treatment as templates, no band was found (lane B in Figure 4). Therefore, it was confirmed that the above-described band was not of the amplified fragment of DNA.

### (Observation of Enzyme Activity of α1,6-FT)

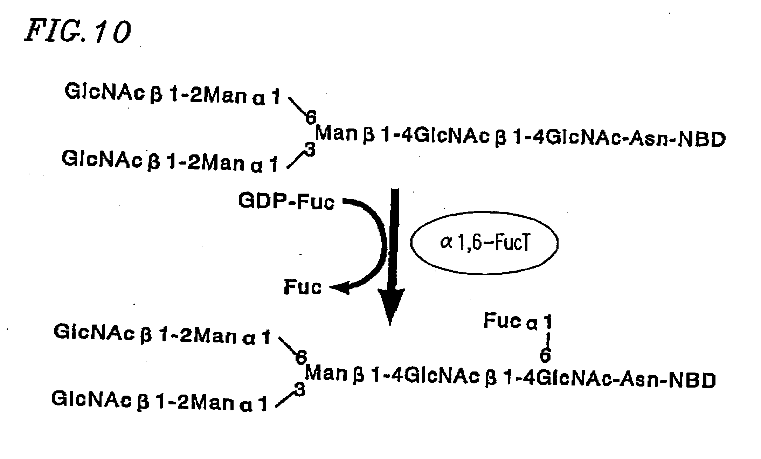

In an α1,6-FT activity measuring kit used in the measurement of α1,6-FT activity in these experiments, a fluorescence-labeled sugar chain having the structure shown in the top of Figure **10** was included as a substrate sugar chain. The fluorescence-labeled sugar chain was prepared in Toyobo Co., Ltd with reference to reports by Yazawa et al. and Seko et al.. (Glycoconj J 1998 Sep; 15(9):863-71 Yazawa S, Kochibe N, Nishimura T, Shima C, Takai I, Adachi M, Asao T, Hada T, Enoki Y, Juneja LR: Biochim Biophys Acta 1997 Apr 17; 1335(1-2):23-32 Seko A, Koketsu M, Nishizono M, Enoki Y, Ibrahim HR, Juneja LR, Kim M, Yamamoto T) as follows. Sugar chains having a linked asparagine residue were prepared from yolk (Gn and Gn-bi-Asn). A fluorescent substance (4-Fluoro-7-nitrohenzofurazan (NBD-F, Dojin Kagaku Kenkyujo)) was attached to the asparagine residue (Gn, Gn-bi-Asn-NBD). Conventionally, a PA sugar chain whose reducing terminal is fluorescence-labeled by 2-aminopyridine is used to measure the activity of sugar transferase. However, in such a PA sugar chain, N-acetylglucosamine at the reducing terminal has an open-loop structure. Therefore, the PA sugar chain cannot serve as a substrate sugar chain for α1,6-FT. Therefore, various acceptor sugar chains and methods have been tried for the measurement of the α1,6-FT activity.

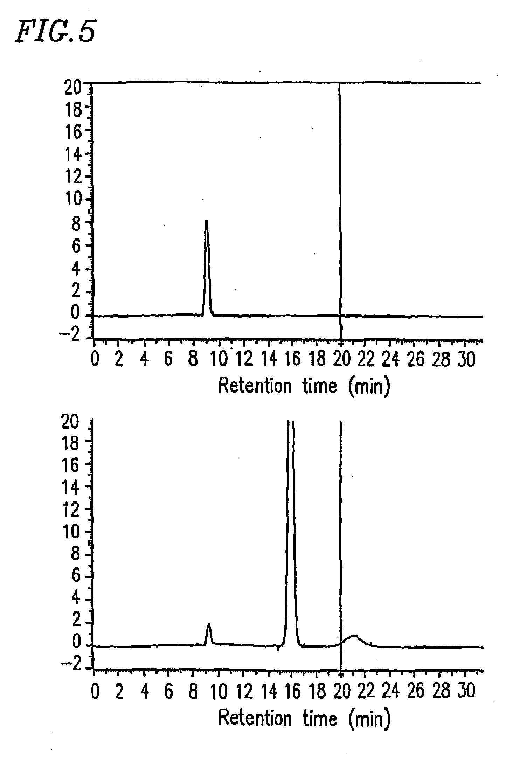

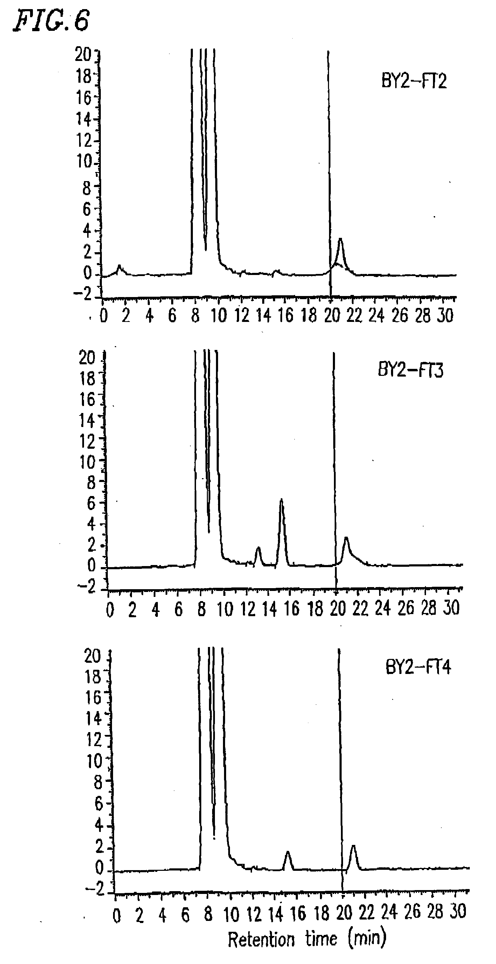

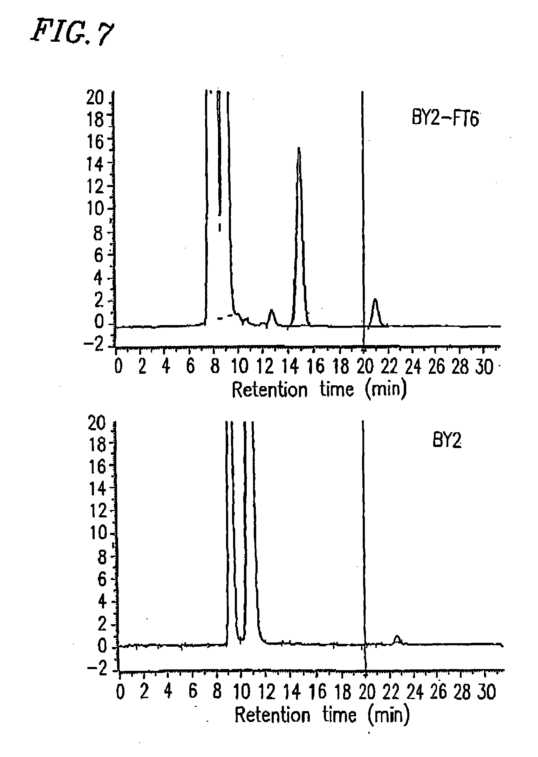

The reaction product was subjected to HPLC analysis under conditions described in the Materials and Methods section 18.3. An unreacted substrate was eluted in about 9.5 minutes (top in Figure 5), while an α1,6-fucosylated sugar chain standard was eluted in about 15 minutes (bottom in Figure **5****).** BY2-FT 2, 3, 4, and 6 for which the expression of mRNA was observed in the RNA-level analysis were studied as follows. A crude enzyme solution was prepared in accordance with the Materials and Methods sections 14 or 18.1. The crude enzyme solution was reacted with the α1,6-FT activity measuring kit. The resultant reaction liquid was subjected to HPLC analysis. As a result, the reaction liquids resulting from the crude enzyme solutions of BY2-FT 3, 4, and 6 exhibited a peak component which was eluted in about 15 minutes (middle and bottom in Figure 6, and top in Figure **7****).** This elution time is the same as that of the α1,6-fucosylated sugar chain standard.

Further, for α1,3-fucosyl transferase (α1,3-FT) existing in plants including a cultured tobacco cell, Studacher et al. (Glycoconj J 1995 Dec; 12(6):780-6 Staudacher E, Dalik T, Wawra P, Altmann F, Marz L;Glycoconj J 1998 Jan; 15(1):89-91 Roitinger A, Leiter H, Staudacher E, Altmann F) has reported that α1,3-FT derived from Mung bean absolutely requires a divalent cation such as Mn²⁺ and Zn²⁺, and does not have activity in the absence of such a cation. No divalent cation was added to the α1,6-FT activity measuring kit and the α1,6-FT crude enzyme solution of this example. In view of this, it is suggested that the peak found in about 15 minutes in the HPLC analysis was not of the α1,3-fucosylated sugar chain. In fact, no peak was found at the position corresponding to 15 minutes in the HPLC chart of a wild-type BY2 specimen (bottom in Figure 7).

### (Measurement of Specific Activity of α1,6-fucosyl transferase)

The specific activity of α1,6-fucosyl transferase was measured for crude protein extract liquids obtained from BY2-FT 3, 4, and 6. The specific activity was evaluated from an HPLC chromatogram in accordance with a method described in the Materials and Methods section 18.4 below. As a result, the specific activity of the non-transformed BY2 strains (indicated by WT in Figure 1) was below the detection limit, while a strain BY2-FT6 exhibited a highest specific activity of 6.03 U/mg protein (Table 1) where 1U is an enzyme amount required to convert 1 pmol of substrate per minute.

**[Table 1]**

| Specific activity of α1,6-FT in crude enzyme solution of BY2-FT | |
|---|---|
| Clone number | Specific activity (U/mg protein) |
| BY2-FT3 | 2.57 |
| BY2-FT4 | 2.53 |
| BY2-FT6 | 6.03 |
| WT | <0.03 |

| | |
|---|---|
| 1U: 1 pmol/min | |

### (Example 2: Influence of α1,6-FT on Glycoprotein in cultured tobacco cell)

Influence of the introduced α1,6-FT on glycoproteins in BY2-FT cells was studied using pea lectin (PSA) which is strongly linked to a fucose residue α1,6 linked to N-acetylglucosamine existing at the reducing terminal of an asparagine-linked type sugar chain. First, a crude protein extract solution was prepared from a BY2-FT cell in accordance with a method described in the Materials and Methods section 14. The approximate value of the crude protein concentration was obtained by measuring absorbance A₂₈₀ (the Materials and Methods section 15). The crude protein extract solution was subjected to SDS-PAGE in accordance with the Materials and Methods sections 16 and 17 below. Thereafter, lectin staining was conducted.

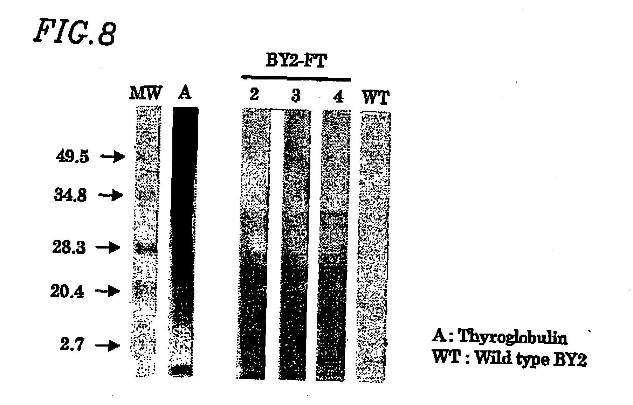

As a result, referring to Figure 8, the glycoprotein sugar chain in the transformant cells exhibited a stain corresponding to about 23 kDa which means a reaction with lectin, as compared to non-transformant BY2 strains (a lane indicated by WT in the right end of Figure 8). This suggests that glycoproteins having an α1,6-fucose residue exist in the BY2-FT 2, 3, and 4 cells. The non-transformed BY2 cell (WT) exhibited slight stain. The reason is consider to be that the PSA has affinity to other fucose residues (including an α1, 3-fucose residue) existing a plant complex-type sugar chain. It should be noted that a lane indicated by A in Figure 8 is a lane obtained by blotting a gel used in electrophoresis of thyroglobulin as a positive control, showing that a reaction with lectin is positive.

### (Example 3: Analysis of Glycoprotein Produced by Transformed Cultured tobacco cells)

Three BY2-FT strains having the highest growth rate were selected. The sugar chain structure of glycoproteins produced by transformed cells having an introduced α1,6-FT gene was analyzed.

### 1. Preparation of Glycoproteins Produced by strain BY2-FT 3

Cultured cells (a wet weight of about 3 kg) of BY2-FT 3 (cultured tobacco cells) were subjected to pulverization with a glass homogenizer, thereby obtaining cell lysates. These cell lysates were centrifuged at 12,000 rpm for 20 minutes at 4°C, thereby obtaining supernatants including glycoproteins. The supernatants were dialyzed with dH₂O (deionized water) 11.5×10⁴-fold dilution) followed by lyophilization, thereby obtaining powdered specimens.

### 2. Preparation of N-linked Sugar Chains

Thereafter, these powdered specimens were subjected to hydrazinolysis at 100°C for 10 hours, thereby cutting out sugar chains from glycoproteins. An excess amount of acetone was added to the hydrazinolysis products, followed by centrifugation at 8,000 rpm at 4° C for 20 minutes, thereby precipitating the sugar chains. A saturated sodium carbonate solution and acetic anhydride were added to the resultant pellet, thereby N-acetylating the sugar chains. Thereafter, the resultant reaction products were subjected to desalination using Dowex 50×2 (Muromachi Kagaku Kogyo). Further, the resultant solutions were applied to TSK gel TOYO PERAL RW-40 (TOSOH) gel filtration column (2.5×30 cm) equilibrated with 0.1 N ammonia solution, thereby recovering N-linked sugar chains.

### 3. Preparation of Pyridylamino (PA) sugar chains

The recovered N-linked sugar chains were converted using 2 aminopyridine to PA sugar chains. The PA sugar chains were purified with TSK gel TOYO PERAL HW-40 (TOSOH) gel filtration column (2.5×30 cm) equilibrated with 0.1 N ammonia solution.

### 4. Fractionation and Analysis of PA sugar chains by HPLC

The structures of the PA sugar chains were analyzed byreversed-phase (RP) HPLC and size-fractionation (SF) HPLC, two-dimensional sugar chain mapping by exo-glycosidase digestion, and MALDI-TOF MS analysis.

The HPLC analysis was conducted using a HITACHI HPLC system equipped with a HITACHI FL Detector L-7480 where the intensity of fluorescence was measured at an excitation wavelength of 310 nm and at a fluorescence wavelength of 380 nm. The RP-HPLC analysis was conducted using a Cosmosil 5C18-P column (6×250 mm; Nacalai Tesque), where the PA sugar chains were eluted by increasing the acetonitrile concentration of a 0.02% aqueous TFA solution from 0% to 6% fox 40 minutes at a flow rate of 1.2 ml/min. The SF-HPLC analysis was conducted using Asahipak NH2P-50 column (4.6×250 mm: Showa Denko), where the PA sugar chains were eluted by increasing the acetonitrile concentration of a dH₂O-acetonitrile mixture from 26% to 50% for 25 minutes at a flow rate of 0.7 ml/min.

The structures of the PA sugar chains were estimated by the two-dimensional sugar chain mapping in which elution times were compared between reversed-phase (RP) HPLC and size-fractionation (SF) HPLC.

### 5. Analysis of PA Sugar Chains by Exo-glycosidase Digestion

The enzyme digestion by N-acetylglycosaminidase (Diplococcus pneumoniae; Roche) was studied as follow. Each PA sugar chain was subjected to reaction in 50 mM sodium acetate buffer (pH 5.45) including 3 mU of N-acetylglycosaminidase at 37°C for two days. The enzyme reaction by α-L-fucosidase (bovine kidney; Sigma) was conducted in 0.1 M sodium acetate buffer (pH 5.45) including 10 mM α-L-fucosidase at 37°C for two days. Each enzyme digestion was arrested by boiling at 100°C for 3 minutes, followed by centrifugation at 12,000 rpm for 5 minutes. The supernatant was subjected to HPLC. The elution times of the specimen sugar chains were compared to the elution times of known sugar chains.

### 6. MALDI-TOF MS analysis

MALDI-TOF MS analysis was conducted using a PerSeptive Biosystems Voyager DE RP Workstation.

### 7. Structures of a PA sugar chain derived from strain BY2-FT3

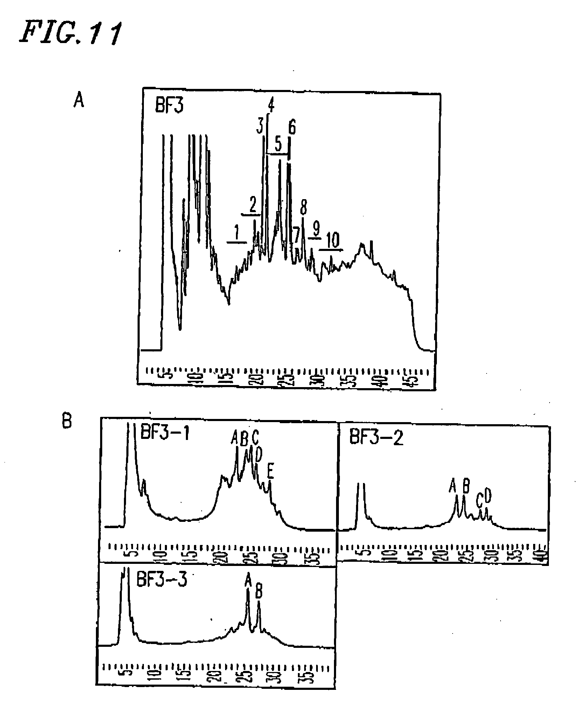

A PA sugar chain prepared from about 3 kg of the BY2 -FT 3, and purified by RP-HPLC and SF-HPLC. Specifically, fractions (1 to 10) obtained by RP-HPLC (see a chromatogram shown in Figure **11A****)** were recovered, and subjected to SF-HPLC. The peaks of fractions 1 to 9 obtained by the RP-HPLC were further subjected to SF-HPLC analysis, resulting in a total of 55 peaks (part of the data is shown in Figure **11B****)**. Some of these peaks included a plurality of PA sugar chains. In this case, such peaks were subjected again to SF-HPLC, thereby purifying the sugar chains thoroughly.

Of the fractions corresponding to the 55 peaks, fractions 4D-V, 5A-III, 5C-III, 5D-II, 6B, 6F-I, and 7E could be cut by fucosidase, and the decomposed products were eluted in RP-HPLC earlier than the intact product (data not shown). This situation indicates that these sugar chains include an α1,6-fucose (Glycoconj J 1998 Jan; 15(1):89-91 HPLC method for the determination of Fuc to Asn-linked GlcNAc fucosyl transferase. Roitinger A, Leiter H, Staudacher E, Altmann F.).

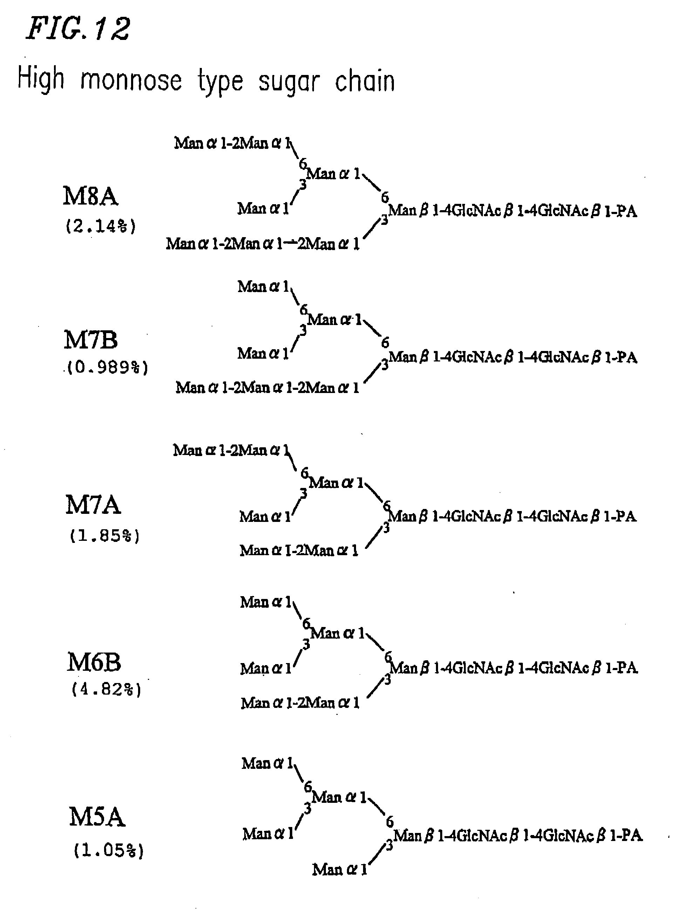

The structure of each sugar chain was analyzed by two-dimensional sugar chain mapping, exo-glycosidase digestion, or MALDI-TOF MS analysis. As a result, the structures of the sugar chains are shown in Figures 12 through 14.

The PA sugar chain of fractions 4D-V, 5C-III, and 5D-II had an m/z of 1413.59 which is substantially equal to that of M3FFX (1413.33). The PA sugar chain treated with fucosidase matched M3FX in the two-dimensional mapping, and had an m/z of 1267.36 which is also substantially equal to that of M3FX (1267.19).

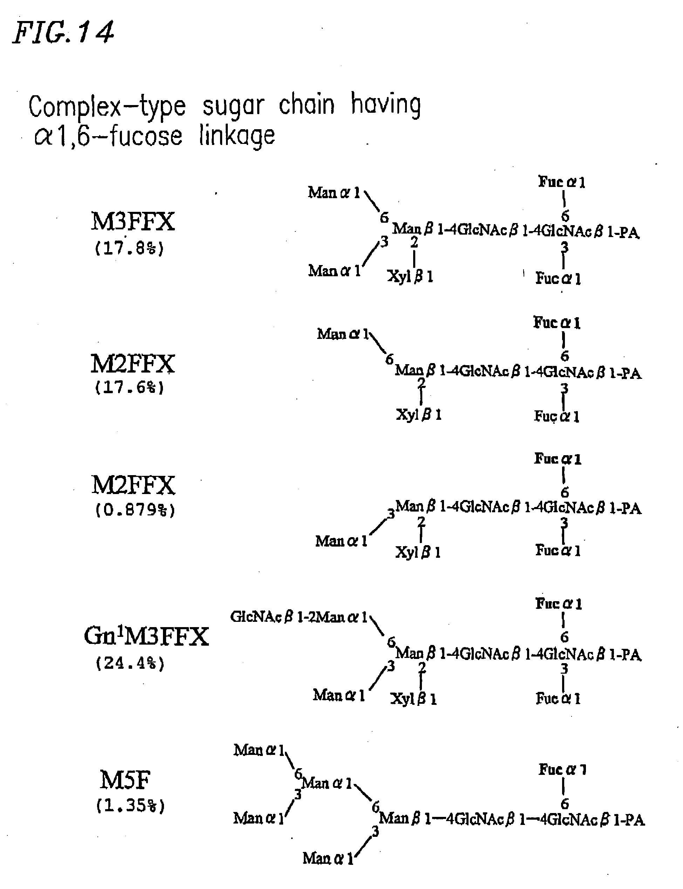

The PA sugar chain of fractions 6B and 5A-III had an m/z of 1251.57 which is substantially equal to that of M2FFX (1251.19) (Figure **14****).** The PA sugar chain treated with fucosidase had an m/z of 1105.79 which is also substantially equal to that of M2FX (1105.05).

The PA sugar chain of fraction 6F-I had an m/z of 1616.14 which is substantially equal to that of Gn¹M3FFX (1616.52) (Figure 14). The PA sugar chain treated with fucosidase matched Gn¹M3FX in the two-dimensional mapping, and had an m/z of 1471.35 which is also substantially equal to that of Gn¹M3PX (1470.38).

The PA sugar chain of fraction 7E had an m/z of 1459.33 which is substantially equal to that of M5F (1459.36) (Figure 14). The PA sugar chain treated with fucosidase matched M5A in the two-dimensional mapping, and had an m/z of 1313.43 which is also substantially equal to that of M5A (1313.22).

The PA sugar chains of fractions 5CII3II and 5DI2II matched M5A in the two-dimensional mapping, and had an m/z of 1313.14 which is substantially equal to that of M5A (1313.22).

The PA sugar chain of fraction 4F matched M6B in the two-dimensional mapping, and had an m/z of 1475.82 which is substantially equal to that of M6B (1475.36).

The PA sugar chain of fraction 3B matched M7B in the two-dimensional mapping, and had an m/z of 1638.35 which is substantially equal to that of M7B (1637.50).

The PA sugar chain of fraction 2C matched M7A in the two-dimensional mapping, and had an m/z of 1638.33 which is substantially equal to that of M7A (1637.50).

The PA sugar chain in peak 1E of fraction 2D matched M8A in the two-dimensional mapping, and had an m/z of 1800.44 which is substantially equal to that of M8A (1475.36).

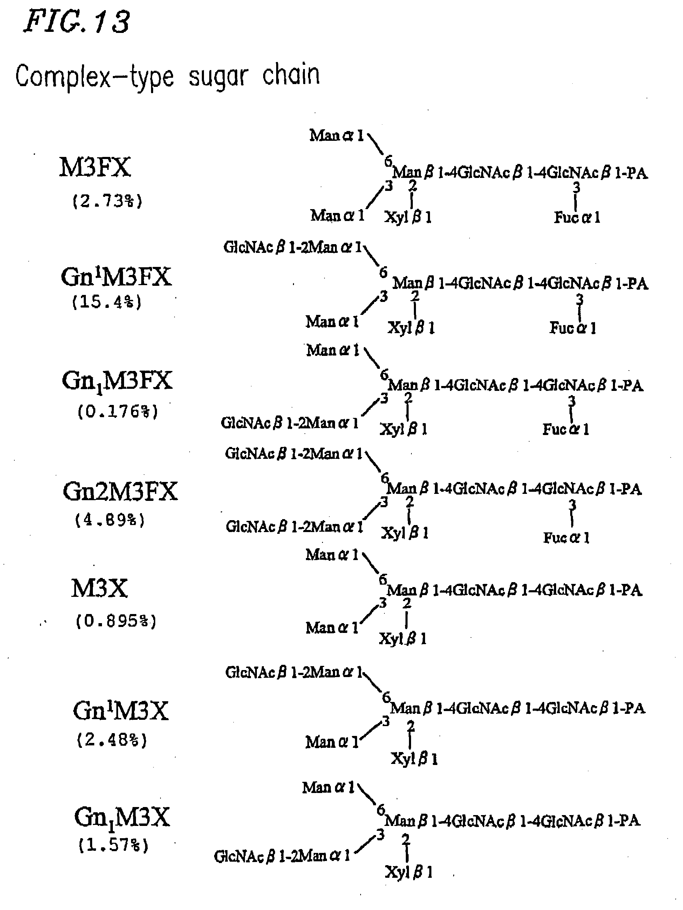

Further, the PA sugar chains of fractions 1AIII and 2A matched M3FX in the two-dimensional mapping. The PA sugar chain of fraction 5CIII matched M3X in the two-dimensional mapping. When the PA sugar chain of fraction 7C was cut with N-acetylglycosaminidase, the elution position of the fragment was shifted by an amount corresponding to one GlcNAcl in the SF-HPLC analysis. The fragment matched M3X in the two-dimensional mapping and had an m/z of 1324.83 which is substantially equal to that of GnM3X (1324.24). Therefore, the PA sugar chain of fraction 7C was considered to be Gn¹M3X. (see Figure 13 for the structure of each sugar chain).

When the PA sugar chains of fractions 5CII2 and 5DI1 were cut with N-acetylglycosaminidase, the elution positions of the fragments were shifted by an amount corresponding to one GlcNAc1 in the SF-HPLC analysis. The fragments each matched M3X in the two-dimensional mapping and had an m/z of 1324.61 which is substantially equal to that of GnM3X (1324.24). Therefore, the PA sugar chains of fractions 5CII2 and 5DI1 were considered to be Gn₁M3X. The elution positions of these PA sugar chains are different from that of the PA sugar chain of fraction 7C in the RP-HPLC analysis. Therefore, it is estimated that these PA sugar chains are structural variants.

When the PA sugar chain of fraction 4EI was cut with N-acetylglycosaminidase, the elution position of the fragment was shifted by an amount corresponding to one GlcNAcl in the SF-HPLC analysis. The fragment matched M3FX in the two-dimensional mapping and had an m/z of 1471.21 which is substantially equal to that of GnM3FX (1470.38). Therefore, the PA sugar chain of fraction 4EI was considered tobe Gn¹M3FX.

When the PA sugar chain of fraction 2BII was cut with N-acetylglycosaminidase, the elution position of the fragment was shifted by an amount corresponding to one GlcNAcl in the SF-HPLC analysis. The fragment matched M3FX in the two-dimensional mapping and had an m/z of 1471.29 which is substantially equal to that of GnM3FX (1470.38). Therefore, the PA sugar chain of fraction 2BII was considered to be Gn₁M3FX. The elution position of this PA sugar chain is different from that of the PA sugar chain of fraction 4EI in the RP-HPLC analysis. Therefore, it is estimated that the PA sugar chain of fraction 2BII is a structural variant.

The PA sugar chain of fraction 3A had an m/z of 1674.56 which is substantially equal to that of Gn2M3FX (1673.57). When the PA sugar chain of fraction 3A was cut with N-acetylglycosaminidase, the elution position of the fragment was shifted by two unit amounts of GlcNAcl in the SF-HPLC analysis. The fragment matched M3FX in the two-dimensional mapping. Therefore, it was estimated that the PA sugar chain of fraction 3A is Gn2M3FX.

In terms of data such as m/z values and two-dimensional mapping results, the other sugar chains did not correspond to any N-linked sugar chains. It was judged that the other sugar chains were not N-linked sugar chains.

As a result of the above-described analyses, the proportions of the N-linked sugar chains are represented by percentages. The high mannose type sugar chain shares 10.8%, the complex-type sugar chain shares 28.1%, and the α1,6-fucose-linked sugar chain shares 61.1%. α1,6-fucose was linked to 61.1% of the sugar chains in BY2-FT 3 transformed by the α1,6-fucosetransferase gene.

As described above, α1,6-fucose was linked to 61.1% of the sugar chains in BY2-FT 3 transformed by the α1,6-fucosetransferase gene. However, α1,3-fucose or β1,2-xylose is also linked to the α1,6-fucose-linked sugar chain. It has been reported that α1,3-fucose or β1,2 -xylose has the possibility of exhibiting antigenicity to animals. To cause such a sugar chain to have a structure having no possibility of exhibiting antigenicity to animals, it is necessary to inactivate α1,3-fucosetransferase or β1,2-xylose transferase. This is achieved by screening or producing a variant host plant having no α1,3-fucosetransferase activity or β1,2-xylose transferase activity, or suppressing gene expression using an enzyme gene.

The suppression of gene expression is achieved by an antisense method (Wenderoth I, von Schaewn A. "Isolation and characterization of plant N-acetylglucosaminyltransferase I (GnTI) cDNA sequences". "Functional analyses in the Arabidopsis cg1 mutant and in antisense plants". Plant Physiol. 2000 Jul; (3):1097-1108), production of a site-specific mutant using a chimeric DNA-RNA oligonucleotide (Beetham PR, Kipp PB, Sawycky XI, Arntzen CJ, May GD. "A tool for functional plant genomics: chimeric RNA/DNA oligosaccharides cause in vivo gene-specific mutations". Proc. Natl. Acad. Sci. USA 1999 Jul ; 96(15):8774-8778), and gene silencing using a plant virus (Covey SN, Al-Kaff NS. "Plant-DNA viruses and gene silencing". Plant Mol Biol 2000 Jun; 43(2-3) :307-322). These techniques are known in the art.

### (Example 4: Production of a plant regenerated from Transformed Tobacco Cell and Analysis of Glycoprotein produced by the Plant)

### 1. Production of Sterile Tobacco Plant

A seed of tobacco (Nicotiana tabacum SR1 (obtained from Leaf Tobacco Laboratory of Japan Tobacco Inc., 700 Higashihara, Toyoda-cho, Iwata-gun, Shizuoka) was placed to a centrifuge microtube of 1.5 ml. 70% ethanol was added to the tube. The tube was shaken for three minutes to sterilize the tobacco seed. Thereafter, the ethanol solution was removed. The seed was washed with 1 ml of sterilized water. Following this, 1 ml of an antiformin solution (a 10-fold dilution of a commercially available sodium hyochlorite solution) was added to the tube which was in turn allowed to stand for 15 minutes while being sometimes shaken. Thereafter, the antiformin solution was removed and the tobacco seed was washed with sterilized water three times.

On the other hand, Augpenin (produced by Meiji Seika Kaisha, Ltd.) was diluted in a petri dish to a final concentration of 160 mg/1. A sterilized filter paper was immersed in the petri dish. The tobacco seed was germinated on the filter paper. The germinated seed was transferred to MS medium, and cultivated in a bright place. The grown tobacco SRI strain plant was cut with a knife about 4 cm under the shoot apex. The cutting of the plant was planted on new MS medium for rooting, and further cultivated in a bright place.

### 2. Transformation of Tobacco Plant

A tobacco plant was transformed in accordance with a method described in An et al. (An, G., Ebert P.R., Mitra A. and Ha S.B. (1988) Binary vectors. In Plant Molecular Biology Manual, A3, 1-19, Academic Dordrecht).

In brief, a sterile tobacco leaf was cut off a plant from a pot. The leaf was cut into about 1 cmxl cm squares (leaf discs) in a petri dish. The leaf discs were transferred to another sterilized petri dish. 5 ml of an Agrobacterium culture medium (Agrabacterium EHA101 having pGPTV-HPT-FT) which had been cultured in a 2xYT medium at 28°C for two days was added to the petri dish and mixed thoroughly, and thereafter allowed to stand for three minutes. The leaf discs were taken out and wiped with a Kim towel to remove the attached excess bacteria liquid. Thereafter, the leaf discs were placed in an MS-NB medium (4.3 g of Murashige-Skoog plant salt mixture, 30 g of sucrose, 10 ml of 5% MES-KOH (pH 5.7), 3 g of gellan gum, 0.1 mg of NAA, 1.0 mg of BAP, 10 mg of thiamin hydrochloride, 1 mg of nicotinic acid, 1 mg of pyridoxin hydrochloride per 1 L of water), and cultivated at 25°C in a bright place.

After two days, the leaf discs were transferred to a 50 ml conical tube Including sterilized water, and washed by shaking thoroughly. After the water content of the leaf discs was wiped off with a Kim towel, the leaf discs were placed in a sterilized medium, and cultivated at 25°C for one week. Thereafter, the leaf discs were transferred to an MS-NB medium (shoot forming medium) including hygromycin B (a final concentration of 20 mg/L) and carbenicillin (a final concentration of 250 mg/L). Grown-up calluses were sterilely planted in glass pots including a shoot forming medium as needed. After about one month, a shoot having a developed stalk and leaves was cut off a plant, and sterilely planted in an MS-NB medium (root forming medium) including hygromycin B (a final concentration of 20 mg/L) and carbenicillin (a final concentration of 250 mg/L) (note that the medium was the same as the above-described basic MS-NB medium except for BAP and NAA), and cultivated at 25°C in a bright place until the shoot grew roots. The plant grown up in the pot was transferred to a bowl, and continued to be grown, thereby obtaining transformed plants FT(1), FT(2), FT1, FT2, and FT3.

### 3. Preparation of Chromosomal DNA from Tobacco Plant

About 100 mg of a plant specimen obtained from each transformed plant FT(1), FT (2), FT1, FT2, and FT3 was frozen in liquid nitrogen. After these frozen specimens were pulverized, chromosomal DNA was prepared from each specimen using DAeasy Plant Mini Kit (QIAGEN) in accordance with instructions thereof.

Thereafter, each chromosomal DNA was subjected to PCR under the conditions similar to those for the amplification of the genomic DNA of the BY2-FG cell described in Example 1. It was confirmed that the α1,6-FT gene was incorporated into a chromosome of the transformed plant. As a primer,CaMV primer (5' -CGTCTTCAAAGCAAGTGGAT) and FT-Sal (5' -GGATATGTGGGGTACTTGAC) were employed.

The resultant PCR amplified products were subjected to electrophoresis similarly to Example 1. The result is shown in Figure **14****.** As shown in Figure **14**, FT(1), FT(2), FT1, FT2, and FT3 exhibited bands around 1700 bp which is considered to indicate an amplified fragment of α1,6-FT gene region. On the other hand, when genomic DNA prepared from a wild-type SR1 was employed as a template, no band was found around 1700 bp. Therefore, it was confirmed that the α1,6-FT gene is incorporated into a chromosome in transformed plants FT(1), FT(2), FT1, FT2, and FT3.

### 4. Analysis of Glycoproteins Produced in α1-6FT Transformed Plant: Lectin staining

Similarly to Example 2, glycoproteins produced by an a1,6-FT-introduced transformant were analyzed using pea lectin (PSA) which is strongly linked to a fucose residue α1,6 linked to N-acetylglucosamine existing at the reducing terminal of an asparagine-linked type sugar chain (Yamamoto K, Tsuji T, Osawa T., (1982) Carbohydrate Res., 110, 283-289, Debray H., Montreuil J., (1989) Carbohydrate Res., 185, 15-26).

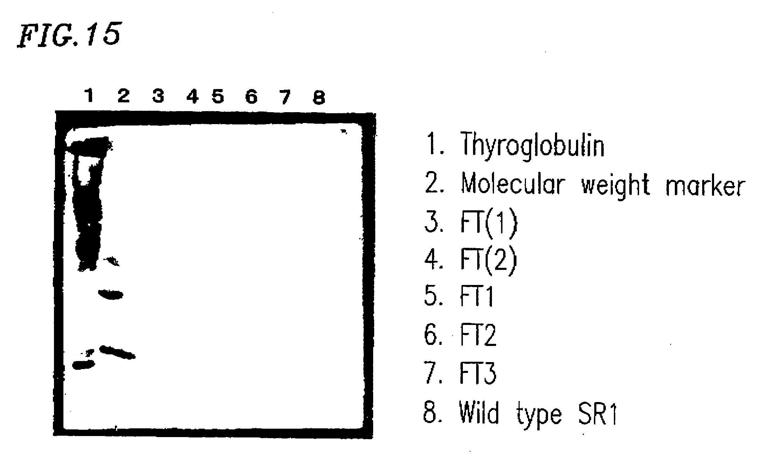

The result is shown in Figure **15**. As shown in Figure **15**, transformed plants FT(1), FT(2), FT1, FT2, and FT3 exhibited staining indicating that lectin reacted with a glycoprotein sugar chain, as compared to an SR1 plant. Therefore, it was shown that glycoproteins having an α1,6-fucose residue exist in transformed plants.

It should be noted that although the SRlplant exhibited a slight level of lectin reaction, such lectin reaction was observed even when cultured tobacco cells were transformed with the α1,6-FT gene and positive clones were screened with PSA lectin. The reason is considered to be that the PSA lectin has an affinity for other fucose residues including an α1,3-fucose residue existing in a plant complex-type sugar chain.

Hereinafter, the materials and methods used in the above-described Examples will be briefly described.

### -Materials and Methods-

### 1. Plants , strains, plasmids

### (1. 1. Plants)

As a plant transformant, a tobacco BY2 cultured cell (Nicotiana tabacum L. cv. Bright Yellow 2) was used.

### (1.2. strains)

Plants used are shown in Table 2.

**[Table 2] Strains**

| Strains | Gene type and characteristic |
|---|---|
| Escherichia coli | |
| JM109 | recA1, endA1, gryA96, thi, hsdR 17, supE 44, relA1, Δ(lac-proAB)/F[traD 36, proAB⁺, lacIq, lacZΔM15] |
| DH5α | supE44, ΔlacU169(Φ801acZΔM15), hsdR17, recA1, endA1, gyrA96, thi-1, relA1 |
| Agrobacteriumu tumefaciens | |
| EHA101 | Kanamycin^{r} Carrying the trans-acting virulence functions necessary to facilitate the transfer of the T-DHA region of binary vectors to plant |
| LBA4404 | Rifampicin^{r}, Streptomycin^{r} |

### (1. 3. Plasmids)

Plasmids used are shown in Table 3.

**[Table 3] Plasimds**

| Plasmids | Gene type and characteristic |
|---|---|
| pUC19 | Amp^{r}, lacZ |
| pHI221 | Amp^{r} |
| | CaMV 35S promoter, GUS gene, and nopaline synthase terminator cloned into pUC19 |
| pGPTV-HPT | Km^{r}, Hm^{r} |
| pGPTV-DHFR | Km^{r}, Methotrexate^{r} |
| pGPTV-BAR | Km^{r}, Bialaphos^{r} |

### 2. Media

### (2. 1. Medium for cultivating bacteria)

2×YT medium: Bacto-tryptone 16 g/l, Yeast extract 10 g/l, andNaCl5 g/l were used. 12 g/l purified agar powder was added to a plate medium. ampicillin (Meiji Seika Kaisha, Ltd.), kanamycin (Meiji Seika Kaisha, Ltd.), hygromycin (Wako Chemicals), chloram phenicol (Wako Chemicals), rifampicin (Wako Chemicals), streptomycin (Wako Chemicals) were optionally added to a final concentration of 50 mg/l, 50 mg/l, 20 mg/l, 25 mg/l, 50 mg/l, and 20 mg/l, respectively.

### (2. 2. Medium for cultured tobacco cell)

**[Table 4]**

| Modified | LS medium | (mg/l) | |
|---|---|---|---|
| NH₄NO₃ | 1650 | CaCl₂·2H₂O | 440 |
| KNO₃ | 1900 | MgSO₄·7H₂O | 370 |
| KH₂PO₄ | 370 | Na₂ ·EDTA | 37.3 |
| H₃BO₃ | 6.2 | FeSO₄·7H₂O | 27.8 |
| MnSO₄·4H₂O | 22.3 | thiamin hy drochloride | 10 |
| ZnSO₄·7H₂O | 8.6 | nicotinic acid | 1 |
| KI | 0.83 | pyridoxin hydr ochloride | 1 |
| Na₂MoO₄·2H₂O | 0.25 | myo-inositol | 100 |
| CuSO₄·5H₂O | 0.025 | sucrose | 30 000 |
| CoCl₂·2H₂O | 0.025 | | |
| | | adjusted by KOH to pH 5.8 | |

Further, mixed salts from Murashge-Skoog medium (Wako Chemicals) were used to prepare the above composition.

### Modified LS agar medium:

The KH₂PO₄ of a modified LS medium was set to 170 mg/l, the pH thereof was adjusted to 5.8 with KOH, and further 3 g/l gellan gum (Wako Chemicals) was added to the medium. Kanamycin, carbenicillin, (Wako Chemicals), hygromycin, methotrexate (Wako Chemicals), and bialaphos (Meiji Seika Kaisha, Ltd.) were optionally added to a final concentration of 150 mg/l, 250 mg/l, 20 mg/l, 0.1 mg/l, and 10 mg/l.

### 3. Reagents and enzymes

Reagents used were obtained from Wako Chemicals and Nacali Tesque, unless otherwise mentioned restriction enzymes and modification enzymes were obtained from Toyobo Co., Ltd., Takara Shuzo Co., Ltd., Nippon Gene, Sigma, and NEB and used in accordance with the directions thereof.

### 4. Transformation of E. coli

### (4. 1. Preparation of Competent Cell)

A host E. coli was inoculated in 2 ml of 2×YT medium, and cultivated overnight. The culture medium was inoculated in 200 ml of 2×YT medium in a Sakaguchi flask. The flask was shaken at 37°C until the turbidity was 0.6 at 600 nm, followed by centrifugation (5,000 rpm, 10 min, 0°C). The supernatant was removed. The bacteria were suspended in 5 ml of a mixture of 50 mM CaCl₂ and 15% glycerol. Thereafter, the suspension was divided into Eppendorf tubes which were preserved as competent cells at -80°C.

### (4. 2. Transformation of E. coli)

The competent cells were thawed on ice. Thereafter, 1 to 15 µl of a DNA solution was added to the competent cells which were in turn allowed to stand in ice for 30 minutes. Thereafter, the solution was allowed to stand at 42°C for 90 seconds, and thereafter immediately returned to ice. 1 ml of 2xYT medium was added to the solution. The competent cells were cultivated at 37°C for one hour, applied to an agar medium including an appropriate antibiotic, and cultivated overnight at 37°C.

### 5. Transformation of Agrobacterium

Agrobacterium was transformed using Bevan et al. 's triparental mating method. In brief, Escherichia coli having a pGPTV-type plasmid and Escherichia coli having a helper plasmid pRK2013 were cultivated at 37°C overnight in a medium including an antibiotic, respectively. Agrobacterium EHA101 or LBA4044 was cultivated at 28°C for two nights in a medium including an antibiotic.

1.5 ml of each culture medium was poured into an Eppendorf tube followed by centrifugation to collect bacteria. The collected bacteria were washed with 2xYT medium twice, and thereafter suspended in 1 ml of 2xYT medium. The three strains were mixed and applied to 2×YT medium, and cultivated at 28°C, so that the plasmids underwent conjugate transfer from the E. coli to the Agrobacterium. Two days later some of the colonies appearing on the 2xYT medium were removed using a platinum loop, and applied to 2xYT agar medium including an antibiotic. After cultivation for two days at 28°C, a single colony was selected.

### 6. Transformation of cultured tobacco cells

### (6. 1. Subculture of cultured tobacco cells)

95 ml of a modified LS medium was poured to a 300 ml Mayer flask. Cultivation was conducted in a dark place at a temperature of 25°C to 27°C while stirring at 120 rpm. 2 ml of cultured cells reaching a stationary phase were subcultured every seven days. When a sufficient amount of cells was not obtained in the seventh day, a double amount (4 ml) of cells were subcultured.

### (6. 2. Transformation of cultured tobacco cells)

100 µl of an Agrobacterium culture solution (Agrobacterium EHA101, LBA4044 including a pGPTV-type plasmid) which had been cultured in 2xYT medium including an antibiotic at 28°C for two days, was well mixed in a petri dish with 4 ml of a suspension of cultured tobacco cells cultured in the fourth day. Thereafter, the'mixture was allowed to stand in a dark place at 25°C. After two days, the suspension in the petri dish was transferred to a centrifuge tube, followed by centrifugation (1,000 rpm, 5 min) to remove the supernatant. A new medium including 250 mg/l carbenicillin was added to the resultant pellet, followed by centrifugation to wash the cells. This wash was repeated three times, so that Agrobacterium was removed. The cultured tobacco cells free from Agrobacterium were applied to a modified LS agar medium including 20 mg/l hygromycin and 250 mg/l carbenicillin, and cultured in a dark place at 25°C. The cells, grown to the callus stage after about two to three weeks, were transferred to new modified LS agar medium to screen growing clones. After a further two to three weeks, the clones, grown to a diameter of one cm, were transferred into 30 ml of modified LS liquid medium including hygromycin and carbenicillin, and subcultured.

### 7. Preparation of small quantity of plasmid DNA

A small quantity of plasmid was obtained from E. coli and Agrobacterium by Birnboin and Doly' s alkaline extraction procedure. Bacteria were cultivated in 2×YT medium including an antibiotic overnight (two nights for Agrobacterium). The medium was transferred to an Eppendorf tube which was in turn centrifuged (12,000 rpm, 5 min, room temperature) to collect the bacteria. The resultant bacteria were suspended in 100 µl of Solution I (in the case of Agrobacterium, 5 mg/ml lysozyme (Sigma) was included), and allowed to stand for 5 minutes at room temperature. Thereafter, 200 µl of Solution II was added to the suspension followed by thorough stirring. The resultant mixture was allowed to stand on ice for 5 minutes. Further, 150 µl of Solution III was added to the mixture followed by thorough mixing. The resultant mixture was allowed to stand in ice for 5 minutes. After centrifugation (12,000 rpm, 5 min, room temperature), the supernatant was transferred to another tube. The supernatant was subjected to RNAse treatment (37°C, 30 min). After extraction with phenol-chloroform, ethanol precipitation was conducted. The resultant pellet was dissolved an appropriate amount of TE buffer

**[Table 5]**

| | |
|---|---|
| TBE buffer: | 12.1 g/l Tris |
| | 6.18 g/l borate |
| | 0.7 g/l EDTA |
| | |
| Gel-Loading buffer: | 0.25% bromophenol blue |
| | 0.25% xylene cyanol |
| | 40%(w/v) sucrose |

### 8. Electrophoresis of DNA

1.0 to 1.5% (w/v) agarose was prepared from TBE buffer. One part of Gel Loading buffer was added to five parts of specimen. The specimens were loaded into the slots of the gel. The electrophoresis apparatus used was Mupid-2 (Cosmobio). Electrophoresis was conducted in I×TBE buffer in the presence of a constant voltage of 100 V. After the electrophoresis, the gel was immersed in a 0. 5 µg/ml aqueous ethidium bromide solution for 20 minutes. The stained gel was placed on a trans-illuminator to be observed.

### 9. Recovery of DNA fragments from the electrophoresis gel

DNA fragments were recovered from the Agarose gel using a Gene clean II kit (Funakoshi). The gel including an intended fragment was transferred to an Eppendorf tube. 1/2 parts of TBE modifier and 4.5 parts of NaI were added to one part of the agarose gel. The mixture was incubated at 55°C to dissolve the gel completely. 5 µl of matrix was added to the mixture followed by thorough mixing. Thereafter, the mixture was allowed to stand on ice for 10 minutes. After brief centrifugation, the supernatant was removed, and the pellet was washed three times with 200 µl of wash buffer. The pellet was dissolved in 6 µl of TE buffer. Thereafter, the solution was subjected to elution at 55°C for 5 to 10 minutes followed by centrifugation. The supernatant included DNA was obtained.

### 10. Preparation of chromosomal DNA from tobacco

### (10.1. Preparation of Chromosomal DNA from cultured tobacco cells)

Chromosomal DNA was prepared from cultured tobacco cells using ISOPLANT (Nippon Gene). 300 µl of Solution I was added to about 0.1 g of cultured tobacco cells, and stirred thoroughly. Further, 150 µl of Solution II was added and thoroughly stirred with a Vortex. The cells were incubated at 50°C for 15 minutes. 150 µlof Solution III was added to the cells, stirred, and allowed to stand for 15 minutes. After centrifugation (12,000 rpm, 15 min, 4°C), the supernatant was subjected to ethanol prepitation two times. The pellet was dissolved in 20 µl of TE buffer, and treated with 1 µl of RNase A (10 mg/ml) for 30 minutes.

### (10. 2. Preparation of chromosomal DNA from tobacco callus)

Chromosomal DNA was prepared from a callus using a DNeasy Plant Mini Prep Kit (QIAGEN). After a callus grown to a diameter of about 1 cm was frozen in liquid nitrogen, the callus was pulverized using a triturator and a pestle to be powder. This powder (100 mg) was used as a specimen to prepare DNA in accordance with the directions of the kit.

### 11. Preparation of all RNAs from tobacco cultured cell callus

All RNAs of a callus were prepared using an RNeasy Mini Prep Kit (QIAGEN). In this case, tritutator, pestle, and sterilized water were treated with 0.05% dimethyl pyrocarbonate, and thereafter autoclaved (120°C, 30 min). After a callus grown to a diameter of about 1 cm was frozen in liquid nitrogen, the callus was pulverized using a triturator and a pestle to powder. This powder (100 mg) was used as a specimen to prepare RNA in accordance with the directions of the kit.

### 12. PCR

### (12.1. Reaction system)

1 µl of Chromosomal DNA, 5 µl of 10×PCR buffer (attached to Takara Ex Taq produced by Takara Shuzo Co., Ltd.), 4 µl of dNTPs (attached to Takara Ex Taq produced by Takara Shuzo Co., Ltd., 2.5 mM), primers (20 pmol each), 0.5 µl of Takara Ex Taq (5 U/µl, Takara Shuzo Co., Ltd.), and sterilized water were mixed to a total volume of 50 µl.

### (12. 2. Reaction conditions)

Reaction was conducted under the following conditions. For the thermal cycler, PCR System 9700 (PE Biosystems) was employed.

**[Table 6]**

| | | |
|---|---|---|
| Stage I: | 1 cycle | Denaturation (94°C) 5 min |
| | | Annealing (60°C) 2 min |
| | | Elongation (72°C) 3 min |
| Stage II: | 30 cycles | Denaturation (94°C) 1 min |
| | | Annealing (60°C) 2 min |
| | | Elongation (72°C) 2 min |
| Stage III: | 1 cycle | Denaturation (94°C) 1 min |
| | | Annealing (60°C) 2 min |
| | | Elongation (72°C) 3 min |

The annealing temperature was changed depending on the Tm of primers used.

### 13. RT-PCR

### (13. 1. Reverse transcription reaction)

Reverse transcription was conducted using RNA PCR Kit Ver.2.1 (Takara Shuzo Co., Ltd.). 4 µl of MgCl₂ (5 mM), 2 µl of 10×RNA PCR buffer, 8.5 µl of RNAse free H₂O, 2 µl of dNTPs (1 mM), 0.5 µl of RNAse Inhibitor (1U/µl), 1 µl of Reverse Transcriptase(0.25U/µl), and 1µl of Oligo dT-Adapter Primer (0.125 µM) attached to the kit, and 1 µl of an RNA specimen prepared as described in the above section 11 were mixed and allowed to react in accordance with a program described below. For the thermal cycler, PCR System 9700 (PE Biosystems) was used where the number of cycles was one, and one cycle includes 50°C 30 minutes; 99°C 5 minutes; and 5°C 5 minutes.

### (13. 2. PCR after reverse transcription reaction)

6 µl of MgCl₂ (2.5 mM), 8 µl of 10×RNA PCR buffer, 63.5 µl of distillation sterilized water, 0.5 µl of TaKaRa Taq (2.5 U/100 µl), Primer (20 pmol) were mixed and added to the tube in which the reverse transcription of the above section 13. 1 had been conducted. After centrifugation by a micro-centrifugal machine for 10 seconds, the tube was allowed to react under the following conditions, Stage I: 1 cycle; 94°C 2 minutes, and Stage II: 45 cycles; 94°C 30 seconds: 60°C 30 seconds; 72°C 1.5 minutes.

### 14. Preparation of crude protein extract solution from cultured tobacco cells

Cultured tobacco cells in the seventh day of subculture were harvested by centrifugation (3,000 rpm, 15 min, 4°C). Thereafter, the obtained cultured tobacco cells were washed with an equal amount of 50 mM sodium phosphate buffer (pH 7.0) by mildly inverting the tube. This process was repeated three times, followed by centrifugation (3,000 rpm, 15 min, 4°C). The harvested cells were transferred to a hand homogenizer (20 ml IKEMOTO) and pulverized. Thereafter, the cell pulverized liquid was transferred to a 50 ml centrifuge tube, followed by centrifugation (12,000 rpm, 20 min, 4°C) to obtain a supernatant which was a crude protein extract solution. One Protease inhibitor cocktail tablet (BOEHRINGER MANNHEIM) was optionally added per 50 ml of the extract liquid. Further, when the enrichment of crude proteins was required, ammonium sulphate (Wako Chemicals) was optionally added to 70% saturation, and allowed to stand on ice for 4 to 5 hours, followed by centrifugation (12,000 rpm, 20 min, 4°C). The resultant proteins were suspended in 500 µl of sterilized water which was used in the subsequent analysis.

### 15. Quantitation of proteins

Proteins were quantitated using DC Protein Assay Kit (Bio-Rad). This kit is based on a Lowly-Folin method. In accordance with the directions thereof, reaction liquids were mixed and allowed to stand at room temperature for 15 min. Thereafter, absorbance was measured at 750 nm. Calibration curves were prepared in the range of 0.05 to 0.4 mg/ml using calf bovine albumin as a standard. The amounts of proteins were determined with reference to the calibration curves.

### 16. Electrophoresis of proteins

### (16. 1. Tris-glycine dodecyl sodium sulfate-polyacrylamide gel Electrophoresis)

Sodium dodecyl sulfate-polyacrylamide gel electrophoresis (SDS-PAGE) was conducted in accordance with Laemmli's method. For the electrophoresis gel. 12.5% polyacrylamide gel was used for separation, and 2.5% polyacrylamide gel (acrylamide : bisacrylamide =30 ; 0.8) was used for enrichment. For the electrophoresis buffer, a Tris-glycine buffer was used. 12 µl of a specimen was heated in a specimen buffer at 100°C for 3 min to be denatured. The electrophoresis was conducted at a constant voltage of 100 V.

### (16. 2. Molecular weight markers)

**[Table 7]**

| For the molecular weight marker, used was Protein molecular weight marker "First" (Daiichi Kagaku Yakuhin) | |
|---|---|
| Phosphorylase | 97,400 |
| Calf bovine albumin | 66,270 |
| Aldolase | 42,200 |
| Carbonic anhydrase | 30,000 |
| Soy bean trypsin inhibitor | 20,100 |
| Lysozyme | 14,000 |
| or, Prestained SDS-PAGE Standar ds (Bio-Rad) | |
| PhosphorylaseB | 106,000 |
| Calf bovine albumin | 80,000 |
| Ovalbumin | 49,500 |
| Carbonic anhydrase | 32,500 |
| Soy bean trypsin inhibitor | 27,500 |
| Lysozyme | 18,500. |

### (16. 3. Staining of proteins)

Coomassie-blue staining and silver staining were conducted. For coomassie-blue staining, gel was immersed for 30 minutes in a staining liquid (0.1% coomassie-brilliant-blue R-250, methanol : acetate : water = 5 : 5 : 2 (v/v) mixture), and thereafter immersed in a bleaching liquid (methanol : acetate : water = 2 : 1 : 7 (v/v) mixture) and shaken overnight. Silver staining was conducted using a silver staining kit (Wako Chemicals) in accordance with a method described in the directions thereof.

### 17. Lectin staining

After SDS-PAGE, the gel was equilibrated in a blotting buffer for 15 minutes. Thereafter, proteins in the gel were blotted on a PVDF membrane (Bio-Rad, Immuno-Blot PVDF Membrane for Protein Blotting, 0.2 mm) at a constant current of 1 mA/am² for 60 to 70 minutes using a semi-dry type blotting apparatus (semi-dry transfer apparatus BE-310 Biocraft). After blotting, the PVDF membrane was immersed in a 0.6% H₂O₂/methanol (v/v) solution, and the endogenous peroxidase of cultured tobacco cells was blocked. After the blocking, the PVDF membrane was washed with a wash buffer (10 min, three times). Thereafter, the membrane was immersed in a wash buffer including 5% Skim Milk, and allowed to mildly react at room temperature for two hours. Similarly, the PVDF membrane was similarly washed with a wash buffer. Thereafter, the PVDF membrane was immersed in a 1,000-fold dilution of peroxidase-labeled PSA lectin (1 mg/ml, EY LABORATORIES. INC.) with the washing buffer, and allowed to react at room temperature for 90 minutes. After the reaction, the membrane was washed in a manner similar to that described above. Thereafter, color development was conducted using a POD immunostain set (Wako Chemicals). The above wash buffer's composition was 10mM Tris-HCl (pH 7.4), 0.15 MNaCl, 0.05% Tween 20.

### 18. Measurement of α1,6-fucosyl transferase activity

### (18. 1. Preparation of crude enzyme solution)

Transformed cultured tobacco cells in the seventh day of cultivation were harvested by centrifugation (3,000 rpm, 20 min, 4°C). Thereafter, the cells were washed with an extraction buffer similarly to above section 14, and harvested. Thereafter, the cells were pulverized using a handy homogenizer, followed by centrifugation (12, 000 rpm. 20 min, 4°C). The supernatant was used as a crude enzyme solution. The above extraction buffer's composition: 20mM Tris-HCl (pH 7.5), 0.175% CHAPS.

### (18. 2. Enzyme reaction of α1,6-fucosyl transferase)

α1,6-activity was always measured in a dark place. A substrate liquid (15 µl) for determining the activity of α1,6-fucosyl transferase, which was provided by Toyobo Co., Ltd., was employed. 5 µl of a crude enzyme solution prepared in above section 19.1 was added to a tube including the substrate liquid, and allowed to react at 37°C for three hours . The enzyme reaction was arrested by boiling for one minute. Immediately after that, the tube was transferred onto ice and allowed to stand for one minute. Further, the ice water was spun off the tube. 80 µl of distilled water was added to the tube followed by centrifugation (12,000 rpm, 1 min, 4°C). 30 µl of the resultant supernatant was subjected to HPLC analysis. The substrate liquid for measuring the activity of α1,6-fucosyl transferase includes per 15 µl, 8 µl of 0.5 M MES/NaOH buffer (pH 7.5), 1 nmole/µl Gn, 1 µl of Gn-bi-Asn-NBD, 2 µl of 5 nmole/ml GDP-Fucose (Wako Chemicals), and 4 µl of MilliQ water. The HPLC system (produced by HITACHI) used includes an interface (L-7000), a fluorescence detector (LaChrom L-7480), a pump (LaChrom L-7100), and a column oven (LaChrom L-7300).

### (18. 3. Presence or absence of enzyme activity)

The presence or absence of enzyme activity was determined by HPLC analysis. As a column, reverse-phase Mightysil RP-18 GP150-4.6 (5µm) (Kanto Kagaku 4.6×150 mm) was employed. A substrate sugar chain used for measurement of α1,6-FT activity was fluorescent labeled so that the substrate sugar chain could be specifically detected by a fluorescence detector (Ex; 470 nm, Em; 530 nm). Further, an α1,6-fucosylated sugar chain standard was prepared as follows. 5 µl of α1,6-fucosyl transferase (40 mU/ml, Toyobo Co., Ltd.) was added to a substrate mixture for measuring the activity of α1,6-fucosyl transferase, and allowed to react at 37°C for 15 minutes in accordance with above section 1. 18.

### (18. 4. Measurement of activity)

An area ratio of a peak of a substrate to a peak of a reaction product obtained by HPLC analysis conducted under conditions below is evaluated. Activity was evaluated as the amount of transferred fucose per 1 mg of crude enzyme solution proteins per minute. The total amount of proteins in a crude enzyme solution was quantitated in such a manner as described in above section 15.

**[Table 8]**

| | |
|---|---|
| Buffer A: | 20 mM acetate-ammonia pH 4.0 |
| Buffer B: | 20 mM acetate-ammonia pH 4.0 - 80% acetonitrile |
| Buffer ratio: | B=5% |
| Mode: | Isocratic |
| Flow rate: | 1 ml/min |
| Column tempe rature: | 55°C |
| Ex: | 470 mm |
| Em: | 530 nm |

### INDUSTRIAL APPLICABILITY

The present invention provides a plant cell having an animal-type sugar chain adding function, a plant regenerated from the plant cell, a method for producing the plant cell, a method for producing an animal type glycoprotein using the plant cell. The glycoprotein produced by the plant cell of the present invention has an animal type sugar chain, so that the glycoprotein is not antigenic to animals, particularly humans. Therefore, the glycoprotein of the present invention is suited for administration to animals including humans.

## Claims

1. A plant cell having an animal-type sugar chain adding function, wherein the plant cell has an introduced gene encoding an enzyme derived from an animal, and the enzyme can transfer a fucose residue to a reducing terminal acetylglucosamine residue of a sugar chain of a glycoprotein.

2. A plant cell according to claim 1, wherein the enzyme) derived from an animal is α1,6-fucosyl transferase.

3. A plant regenerated from a plant cell according to claim 1 or 2.

4. A method for producing a plant cell having an animal-type sugar chain adding function, comprising the step of introducing into the plant cell a gene encoding an enzyme derived from an animal, wherein the enzyme can transfer a fucose residue to a reducing terminal acetylglucosamine residue of a sugar chain of a glycoprotein.

5. A method for producing a glycoprotein having an animal-type sugar chain, comprising the steps of:
transforming a plant cell by introducing into the plant cell a gene encoding an enzyme derived from an animal and a gene encoding an exogenous glycoprotein, wherein the enzyme can transfer a fucose residue to a reducing terminal acetylglucosamine residue of a glycoprotein, and
cultivating the resultant transformed plant cell.

6. A method for producing a glycoprotein having an animal-type sugar chain, comprising the steps of:
transforming a plant cell by introducing into the plant cell a gene encoding an enzyme capable of transferring a fucose residue to a reducing terminal acetylglucosamine residue and a gene encoding an exogenous glycoprotein, and
expressing the enzyme in a cell organelle.

7. A glycoprotein having an animal-type sugar chain produced by a method according to claim 6.
